# EUROPEAN PATENT SPECIFICATION

(11) **EP 3 004 376 B1**
(45) Date of publication and mention of the grant of the patent: **09.05.2018**
(21) Application number: 13729200.9
(22) Date of filing: 04.06.2013
(51) Int. Cl.: C12Q 1/68

(54) **METHODS FOR DIAGNOSING CHRONIC VALVULAR DISEASE**
VERFAHREN ZUR DIAGNOSE VON CHRONISCHEN KLAPPENKRANKHEIT
METHODE DE DIAGNOSTIC DE LA VALVULOPATHIE CHRONIQUE

(30) Priority: 03.06.2013 US 201361830380 P
(43) Date of publication of application: 13.04.2016
(73) Proprietor: Nestec S.A., 1800 Vevey (CH)
(72) Inventor: LI, Qinghong, Chesterfield, MO 63305 (US); LAFLAMME, Dorothy, P., Floyd, VA 24091 (US); HANNAH, Steven, S., Chesterfield, MO 63017 (US)
(74) Representative: Krishnan, Sri
(86) International application number: PCT/US2013/044011
(87) International publication number: WO 2014/196957

(56) References cited:
- US-A1- 2006 160 112
- OYAMA MARK A ET AL: "Genomic expression patterns of mitral valve tissues from dogs with degenerative mitral valve disease", AMERICAN JOURNAL OF VETERINARY RESEARCH, vol. 67, no. 8, August 2006 (2006-08), pages 1307-1318, XP009172044, ISSN: 0002-9645
- SHAHRARA SHIVA ET AL: "Upregulation of thyroid hormone receptor beta1 and beta2 messenger RNA in the myocardium of dogs with dilated cardiomyopathy or chronic valvular disease", AMERICAN JOURNAL OF VETERINARY RESEARCH, vol. 60, no. 7, July 1999 (1999-07), pages 848-852, XP009172043, ISSN: 0002-9645
- AUPPERLE H ET AL: "Expression of Genes Encoding Matrix Metalloproteinases (MMPs) and their Tissue Inhibitors (TIMPs) in Normal and Diseased Canine Mitral Valves", JOURNAL OF COMPARATIVE PATHOLOGY, ACADEMIC PRESS, LONDON, GB, vol. 140, no. 4, 1 May 2009 (2009-05-01), pages 271-277, XP026074436, ISSN: 0021-9975, DOI: 10.1016/J.JCPA.2009.01.001 [retrieved on 2009-03-12]
- MAGDALENA LÓJ ET AL: "Genomic and genetic aspects of heart failure in dogs - A review", ACTA VETERINARIA HUNGARICA, vol. 60, no. 1, 25 February 2012 (2012-02-25), pages 17-26, XP055076581, ISSN: 0236-6290, DOI: 10.1556/AVet.2012.002
- M Schwemmer ET AL: "AF143503", Getentry database, 1 September 1999 (1999-09-01), XP055076511, Retrieved from the Internet: URL:http://getentry.ddbj.nig.ac.jp/getentr y/na/AF143503/?filetype=html [retrieved on 2013-08-26]
- MICHAEL SCHWEMMER ET AL: "Assembly and Characterization of Canine Heart Endothelial Nitric Oxide Synthase cDNA and 5'-Flanking Sequence by Homology (RT-)PCR Cloning", NITRIC OXIDE, vol. 3, no. 3, 1 June 1999 (1999-06-01), pages 254-264, XP055076514, ISSN: 1089-8603, DOI: 10.1006/niox.1999.0234
- CHRISTOPHER ORTON E ET AL: "Signaling pathways in mitral valve degeneration", JOURNAL OF VETERINARY CARDIOLOGY, ELSEVIER, NL, vol. 14, no. 1, 8 December 2011 (2011-12-08), pages 7-17, XP028468405, ISSN: 1760-2734, DOI: 10.1016/J.JVC.2011.12.001 [retrieved on 2012-01-21]
- MOESGAARD SOPHIA G ET AL: "Increased nitric oxide release and expression of endothelial and inducible nitric oxide synthases in mildly changed porcine mitral valve leaflets.", THE JOURNAL OF HEART VALVE DISEASE JAN 2007, vol. 16, no. 1, January 2007 (2007-01), pages 67-75, XP009172055, ISSN: 0966-8519
- L.H. OLSEN ET AL: "Increased NADPH-Diaphorase Activity in Canine Myxomatous Mitral Valve Leaflets", JOURNAL OF COMPARATIVE PATHOLOGY, vol. 129, no. 2-3, 1 August 2003 (2003-08-01), pages 120-130, XP055076557, ISSN: 0021-9975, DOI: 10.1016/S0021-9975(03)00019-7

## Description

### BACKGROUND OF THE INVENTION

### Field of the Invention

The invention relates generally to methods for diagnosing chronic valvular disease and particularly to methods for diagnosing chronic valvular disease by measuring gene expression markers associated with chronic valvular disease.

### Description of Related Art

Cardiac disease is one of the most common disorders in dogs. Approximately 11% of dogs suffer cardiac disease, of which have adult onset. One third of dogs ages 10 or over has Chronic Valvular Disease (CVD), CVD is characterized by a progressive degeneration and deformation of the atrioventricular valves, most commonly the mitral valves, resulting in early mitral valve insufficiency. This in turn leads to the appearance of a systolic heart murmur due to mitral regurgitation, whereun inadequate closure of the mitral valve causes blood to flow back to the left atrium. The affected dogs finally develop left atrioventricular volume overload, pulmonary edema, atrial dilatation and supraventricular arrhythmias.

Although surgical or medical treatment of affected valves is possible, nutritional intervention is preferred by pet owners. Early detection and treatment are imperative however detection can be difficult due to the lack of symptoms. Biomarkers are useful for detecting conditions when an animal is displaying minimal symptoms or asymptomatic. Gene expression markers are useful biomarkers. Currently there are no known biomarkers useful as diagnostic agents to measure chronic valvular disease in animals.

Oyama MA et al disclose 229 transcripts differentially expressed in mitral valve samples of dogs with degenerative mitral valve disease, including NOS3 and PAI-1 ("Genomic expression patterns of mitral valve tissues from dogs with degenerative mitral valve disease", Am J Vet Res., 2006 Aug, vol. 67(8), p. 1307-18).

Lój M et al disclose several transcripts differentially expressed in left ventricule samples of dogs with endocardiosis, including MMP9 ("Genomic and genetic aspects of heart failure in dogs - a review.", Acta Vet Hung., 2012 Mar, vol. 60(1): p. 17-26).

Therefore there remains a need for diagnosing and predicting chronic valvular disease in animals to provide the most appropriate and effective level of treatment. Such treatment will improve the animal's quality of life. The present invention satisfies this need.

### SUMMARY OF THE INVENTION

It is, therefore, an object of the present invention to provide methods for diagnosing chronic valvular disease in animals.

This and other objects are achieved using methods for diagnosing chronic valvular disease in an animal, as defined in the claims that involve analyzing a sample from the animal for the presence of gene expression markers associated with chronic valvular disease; comparing the amount of gene expression markers identified in the sample to a corresponding amount of the same gene expression present in a sample from one or more comparable control animals that do not suffer from chronic valvular disease; and using said comparison to diagnose chronic valvular disease in the animal if expression markers found in the animal's sample is differentially expressed in the control animal's sample.

Other and further objects, features, and advantages of the present invention will be readily apparent to those skilled in the art.

### DETAILED DESCRIPTION OF THE INVENTION

### Definitions

The term "animal" means any animal susceptible to or suffering from chronic valvular disease, including human, avian, bovine, canine, equine, feline, hierine, lupuie, murine, ovine, or porcine animals.

The term "differential expression" or "differentially expressed" means increased or upregulated gene expression or means decreased or downregulated gene expression as detected by the absence, presence, change in the amount of transcribed messenger RNA or translated protein in a sample, or means an increase or decrease in the amount of protein present in a sample.

The term "comparable control animal" means an animal of same species and type an individual animal evaluated at two different times.

The term "companion animals" means domesticated animals such as dogs, cats, birds, rabbits, guinea pigs, ferrets, hamsters, mice, gerbils, pleasure horses, cows, goats, sheep, donkeys, pigs, and more exotic species kept company, amusement psychological support, extrovert display, and all of the other functions that humans need to share with animals of other species.

The term "diagnosing" means determining if an animal is suffering from or predicting if the animal is susceptible to developing chronic valvular disease.

As used herein, ranges are used herein in shorthand, so as to avoid having to list and describe each and every value within the renge. Any appropriate value within the range can be selected, where appropriate, as the upper value, lower value, or the terminus of the range.

As used herein, the singular form of a word includes the plural, and vice versa, unless the context clearly dictates otherwise. Thus, the references "a", "an", and "the" are generally inclusive of the plurals of the respective terms. For example, to "a method" includes a plurality of such "methods." Similarly, the words "comprise", "comprises", and "comprising" are to be interpreted inclusively rather than exclusively. Likewise terms "include". "including" and "or" should all be construed to be inclusive, unless such a construction is clearly prohibited from the context.

The methods and compositions and other advances disclosed here are not limited to particular methodology, protocols, and reagents described herein because, as the skilled artisan will appreciate, they may vary. Further, the terminology used herein is for the purpose of describing particular embodiments only, and is not intended to, and does not, limit the scope of that which is disclosed or claimed.

Unlsess defined otherwise, all technical and scientific terms, terms of art, and acronyms used herein have the meanings commonly understood by one of ordinary skill in the art in the field(s) of the invention, or in the field(s) where the term is used.

The discussion of those references is intended merely to summarize the assertions made therein. No admission is made that any such patents, patent applications, publications or references, or any portion thereof, are relevant, material, or prior art. The right to chalenge the accuracy and pertinence of any assertion of such parents, patent applications, publications, and other references as relevant, material, al, or prior art is specifically reserved.

### The Invention

The invention provides methods for diagnosing chronic valvular disease in an animal as defined in the claims.

In one aspect, the disclosure provides methods for diagnosing chronic valvular disease in an animal. The methods comprise obtaining a biological sample from the animal; analyzing the sample for the presence of one or more gene expression markers associated with chronic valvular disease; comparing the amount of gene expression markers identified in the sample to a corresponding amount of the same gene expression markers present in a sample from one or more comparable control animals that do not suffer from chronic valvular disease; and using said comparison to diagnose chronic valvular disease in the animal if the gene expression markers found in the animal's sample are differentially expressed in the control animal's sample.

In one aspect the disclosure provides methods for diagnosing chronic valvular disease in an animal. The methods comprise obtaining a biological sample from the animal; analyzing the sample for the presence of one or more gene expression markers associated with chronic valvular disease; comparing the amount of gene expression markers idenified in the sample to a corresponding amount of the same expression markers present in a sample from one or more comparable control animals that do not suffer from chronic valvular disease; and using said comparison to diagnose chronic valvular desease in the animal if the gene expression markers found in the animal's sample are greater than the amount present in the control animal's sample.

In one aspect, the disclosure provides methods for diagnosing chronic valvular disease in an animal. The methods comprise obtaining a biological sample from the animal; analyzing the sample for the presence of one or more gene expression markers associated with chronic valvular disease; comparing the amount of gene expression markers identified in the sample to a corresponding amount of the same gene expression markers present in a sample from one or more comparable control animals that do not suffer from chronic valvular disease; and using said comparison to diagnose chronic valvular disease in the animal if the gene expression markers found in the animal's sample are less than the amount present in the control animal's sample.

The invention is based upon the discovery that the gene expression markers of the invention are present in the biological sample of an animal and that the amount of the gene expression markers in the sample serves as a biochemical indicator for diagnosing chronic valvular disease by indicating or predicting the threshold for chronic valvular disease. The invention allows veterinary and other clinicians to perform tests for these "biomarkers" in a sample and determine whether the animal is susceptible to or suffering from chronic valvular desease and whether there is a need for further diagnostics or treatment. Having established the need for further diagnostics or treatments, the cost and risk of such further diagnostics or treatments are justified.

In various embodiments, one or more comparable control animals that are not the animal being evaluated for chronic valvular desease and that have been determined not to suffer from chronic valvular disease are evaluated for at least one of the gene expression markers and the results of such evaluations are used as a baseline value for comparison with the results from an animal being evaluated for one or more of the gene expression markers. In preferred embodiments, the baseline value for the gene expression markers is determined by evaluating numerous comparable control animals.

In another embodiment, the amount of at least one of the gene expression markers are determined for an animal at various times throughout the animal's life and the results used to determine if the animal is susceptible to or suffering from chronic valvular disease, *e.g*., if the amount of at least one of the gene expression markers increases or decreases as the animal ages, the animal can be diagnosed as susceptible to or suffering from chronic valvular disease. In preferred embodiments, the animal is evaluated periodically and the results for the gene expression markers are recorded. Then, if a subsequent evaluation shows that the amount of one or more gene expression markers has increased or since the last evaluation(s), the animal is diagnosed as susceptible to or suffering from chronic valvular disease.

Any sample that is of biological origin may be useful in the present disclosure. Examples include, but are not limited to, blood (serum/plasma), celebral spinal fluid (CSF), urine, stool, breath, saliva, or biopsy of any tissue. In one aspect the sample is a tissue sample. In another aspect, the sample is cardiac tissue. In one embodiment the tissue is mitral valve tissue. In another embodiment, the tissue is left ventricle tissue. In one aspect, the sample is a serum sample. While the term "serum" is used herein, those skilled in the art will recognize that plasma or whole blood or a sub-fraction of whole blood may also be used.

In various embodiments of the invention, changes in gene expression may be measured in one or both of two ways: (1) measuring transcription through detection of mRNa produced by a particular gene; and (2) measuring translation through detection of protein produced by a particular transcript.

Decreased or increased expression can be measured at the RNA level using any of the methods well known in the art for the quantitation of polynucleotides, such as, for example, PCR (including, without limitation, RT-PCR and qPCR), RNase protection, Northern blotting, microarray, macroarray, and other hybridization methods The genes that are assayed or interrogated according to the invention are typically in the form of mRNA or reverse transcribed mRNA. The genes may be cloned and/or amplified. The cloning itself does not appear to bias the representation of genes within a population. However, it may be preferable to use polyA+ RNA as a source, as it can be used with fewer processing steps.

Decreased or increased expression can be measured at the protein level using any of the methods well known in the art for protein quantitation, such as, for example, western blotting. ELISA, mass spectrometry, etc.

While the use of one of the gene expression markers is sufficient for diagnosing chronic valvular disease, the use of one or more, two or more, three or more, or four or more of such gene expression markers is encompassed within the disclosure and may be preferred in many circumstances. The gene expression markers can be evaluated and used for a diagnosis in any combination.

In one aspect, the diagnosis is based upon determining the amount of one or more gene expression markers in the animal's sample is selected from NOS3, COL6A5 (COL29A1), Serpine1 (PAI-1), SELP, SLC27A6, EDN1_CANFA, CD74, , MYC, MT2_CANFA, IL8_CANFA, IL6, ACSL1, ADIPOQ, and AGT.

In one embodiment, the diagnosis is based upon determining if the gene expression markers found in the animal's mitral valve sample are greater compared to the amount present in the control animal's sample, wherein the gene expression markers are NOS3, COL6A5 (COL29A1) Serpine1 (PAI-1), SELP, SLC27A6, EDN1_CANFA, CD74, , MYC, MT2_CANFA, IL8_CANFA, and IL6.

In one aspect, the diagnosis is based upon determining if the gene expression markers found in the animal's sample are less than compared to the amount present in the control animal's sample, wherein the gene expression markers are ACSL1, ADIPOQ, and AGT.

In one aspect, the diagnosis is based upon determining the amount of one or more gene expression markers in the animal's mitral valve sample is selected from NOS3, COL6A5 (COL29A1), Serpine1 (PAI-1), SELP, ACSL1, ADIPOQ, O3FAR1, FABP4, SLC27A6, EDN1_CANFA, CD74, GSTP1, MGST1, MYC, MT2_CANFA, IL8_CANFA, IL6, PLIN1, CLDN 1, and AGT.

In one aspect, the diagnosis is based upon determining the amount of one or more gene expression markers in the animal's Left Ventricle sample is selected from NOS2, NOS3, MMP15, MMP8, MMP9, TIMP1, NPPA, COL14A1, HOPX, Serpine1 (PAI-1) ,TGFB3, LIPE, MLYCD, FADS1, ACSF1, LOX, TGFBR2, WNT9B, WNT5A, WISP2, FZD8, OSMR, OSM. ELOVL7, ACOT1, MT2_CANFA, MT1_CANFA, STAT3, EDNRB, TAGLN2 , ADIPOQ, RETN, PLA2G5, PLA2G4A, FFAR2, AGT, NFKBIA, TLR4, FOS, JUNB, AQP9, SOAT1, SMAD6, EDNRB, NKX2-5, GATA4_CANFA, PTGS2 (COX-2), IL8_CANFA, IL6, and MYH1_CANFA.

In one embodiment, the diagnosis is based upon determining if the gene expression markers found in the animal's mitral valve sample are greater compared the amount present in the control animal's mitral valve sample, wherein the gene expression markers are NOS3, COL6A5 (COL29A1), Serpinel (PAI-1), SELP, SLC27A6, EDN1_CANFA, CD74, MYC, MT2_CANFA, IL8_CANFA, IL6, and CLDN1. In a preferred embodiment, the diagnosis is based upon determining if the gene expression markers found in the animal's mitral valve sample are greater compared to the amount present in the control animal's mitral valve sample, wherein the gene expression markers are NOS3, COL6A5 (COL29A1), Serpinel (PAI-1), and SELP.

In one aspect, the diagnosis is based upon determining if the gene expression markers found in the animal's left ventricle sample are greater compared to the amount present in the control animal's left ventricle sample, wherein the gene expression markers are NOS3. MMP15, MMP8, MMP9, TIMP1, NPPA, HOPX, Serpinel (PAI-1), LOX, TGFBR2, WNT9B, OSMR, OSM, ELOVL7, MT2_CANFA, MT1_CANFA, STAT3, EDNRB, TAGLN2, RETN, FFAR2, NFKBIA, TLR4, FOS, JUNB, AQP9, SOAT1, SMAD6, EDNRB, PTGS2 (COX-2), IL8_CANFA, and IL6. In a preferred aspect, the diagnosis is based upon determining if the gene expression markers found in the animal's left ventricle sample are greater compared to the amount present in the control animal's left ventricle sample, wherein the gene expression markers are NOS3, MMP15, MMP8, MMP9, TIMP1, NPPA, HOPX, Serpinel (PAI-1), LOX, TGFBR2, WNT9B, OSMR, OSM, ELOVL7, MT2_CANFA, MT1_CANFA, STAT3, EDNRB, and TAGLN2.

In one aspect, the diagnosis is based upon determining if the gene expression markers found in the animal's mitral valve sample are less than compared to the amount present in the control animal's mitral valve sample, wherein the gene expression markers are ACSL1, ADIPOQ, O3FAR1, FABP4, GSTP1, MGST1, PLIN1, and AGT, In a preferred aspect, the diagnosis is based upon determining if the gene expression markers found in the animal's mitral valve sample are less than compared to the amount present in the control animal's mitral valve sample, wherein the gene expression markers are.

In one aspect, the diagnosis is based upon determining if the gene expression markers found in the animal's left ventricle sample are less than compared to the amount present in the control animal's left ventricle sample, wherein the gene expression markers are NOS2, COL14A1, TGFB3, LIPE, MLYCD, FADS1, ACSL1, WNT5A, WISP2, FZD8, ACOT1, ADIPOQ, PLA2G5, PLA2G4A, AGT, NKX2-5, GATA4_CANFA, and MYH1_CANFA. In a preferred aspect, the diagnosis is based upon determining if the gene expression markers found in the animal's left ventricle sample are than compared to the amount present in the control animal's sample, the gene expression markers are NOS2, COLI4A1, TGFB3, LIPE, MLYCD, FADS1, ACSL1, WNT5A, WISP2, FZD8, and ACOT1. In a more preferred aspect, the diagnosis is based upon determining if the gene expression markers found in the animal's left ventricle sample are less than compared to the amount present in the control animal's sample, wherein the gene expression markers are NOS2, COLI4A1, TGFB3, LIPE, MLYCD, FADS1, and ACSL1.

In various embodiments, the animal is a human or companion animal. Preferably, the companion animal is a canine such as a dog or a feline such as a cat.

### EXAMPLES

The invention can be further illustrated by the following examples, although it will be understood that these examples are included merely for purposes of illustration and are not intended to limit the scope of the invention unless otherwise specifically indicated.

### Example 1

Biomarkers were identified through a differential gene expression profiling study comparing diseased and normal cardiac tissues,

Study Design. Mitral valve necropsies were collected from 3 nondiseased dogs and 3 diseased dogs, while left ventricle necropsies were collected from 4 nondiseased dogs and 2 diseased dogs. Echocardiograms were utilized to classify disease status. RNA sample was extracted from each of necropsy samples, and was sequenced using RNA-seq technology to quantitate the global gene expression levels. Differentially expressed genes (DEG) were also determined.

Results. The study identified eight hundred and twelve (812) differentially expressed genes in left ventricle tissues (Table 5), and two hundred and sixty-three (263) differentially expressed genes in mitral valve tissues (Table 6). Genes were further selected and ranked based on their biological relevance in cardiomyopathy and Chronic Valvular Disease (CVD). A list of top 50 left ventricle genes (Table 1), and a list of top 20 mitral valve genes (Table 3) were generated.

The top 50 left ventricle gene expression markers differentially expressed in left ventricle tissue are identified in Table 1.

**Table 1**

| **Gene name** | **P value** | **FDR** | **Fold change** | **Up/Dn** | **Description** |
|---|---|---|---|---|---|
| NOS2 | 0.000223 | | 21 | Dn | Nitric Oxide Synthase, inducible |
| | | 0.01 | | | |
| NOS3 | 403E-09 | 1.28E-06 | 6 | Up | Nitric Oxide Synthase, endothelial |
| MMP15 | 0.00016 | 0.0087 | 3.3 | Up | matrix tile 15 |
| MMP8 | 0.000366 | 0.016 | 162 | Up | matrix metallopeptidase 8 (neutrophil collagenase) |
| MMP9 | 0.002 | 1 | 9999 | Up | matrix metalloproteinase-9 precursor (gelatinase B, 92kDa gelatinase, 92kDa type IV collagenase) |
| TIMP1 | L03E-06 | 0.0001 | 48 | Up | mettalloproteinase inhibitor 1 precursor |
| NPPA | 0.0002 | 0.012 | 4 | Up | Natriuretic peptides A Atrial natriuretic factor |
| COL14A1 | 0.0012 | 0.04 | 2.67 | Dn | collagen, type XIV, alpha 1 |
| HOPX | 0.003 | 0.11 | 4.67 | Up | HOP homeobox |
| Serpinel (PA1-1) | 0 | 0 | 30 | Up | SERPINE1 serpin peptidase inhibitor, clade E (nexin, plasminogen activator inhibitor type 1). member 1 |
| TGFB3 | 0.007 | 0.14 | 2.5 | Dn | transforming growth factor, beta 3 |
| L1PE | 0.002 | 0.057 | 2.73 | Dn | lipase, hormone-sensitive |
| MLYCD | 00008 | 0.03 | 2.68 | Dn | malonyl-CoA decarboxylase |
| FADS1 | 0.002 | 0.06 | 2.87 | Dn | fatty acid desaturase 1 |
| ACSL1 | 0.001 | 0.046 | 3 | Dn | acyl-CoA synthetase long-chain family member 1 |
| LOX | 2.44E-07 | 4.32E-05 | 5.6097248 | Up | lysyl oxidase |
| TOFBR2 | 0.001158 | 0.04 | 2.5840787 | Up | transforming growth factor, beta receptor 11 (70/80kDa) |
| WNT9B | 1.51E-08 | 3.99E-06 | 13.34 | Up | wingless-type MMTV integration site family, member 9B |
| WNT5A | 2.41E-06 | 0.000277 | 6.96 | On | Protein Wnt-5a [Source:UniProtKB/Swiss-Prot;Acc:Q9QXQ7] |
| WISP2 | 0.000106 | 0.0063 | 3.4 | Dn | WNT1 inducible signaling pathway protein 2 [Source:HGNC Symbol; Acc:12770] |
| FZD8 | 9.21E-06 | 0.000811 | 5.4 | Dn | frizzled family receptor 8 |
| OSMR | 1.46E-08 | 3.94E-06 | 5.4 | Up | oncostatin M receptor |
| OSM | 3.94E-05 | 0.002829 | 41.6 | Up p | oncostatin M |
| ELOVL7 | 0,000118 | 0.006733 | 3.6 | Up | ELOVL fatty acid elongase 7 |
| ACOT1 | 8.63E-05 | 0.005357 | 5.58 | Dn | acyl-CoA thioesterase 1 [Source:HGNC Symbol;Acc:33128] |
| MT2 CANFA | 0 | 0 | 133.4 | Up | Metallothionein-2 |
| MT1 CANFA | 3.55E-15 | 3.77E-12 | 24.1 | Up | Metallothionein-1 |
| STAT3 | 4.72E-05 | 0.003281 | 3.45 | Up | signal transducer and activator of transcription 3 (acute-phase response factor) |
| EDNRB | 8.66E-06 | 0.000796 | 3.66 | Up | endothelin B receptor precursor |
| TAGUN2 | 0.00031 | 0.014471 | 2.8 | Up | transgelin 2 [Source:HGNC Symbol;Acc:11554] |
| AD1POQ | 5.96E-09 | 1.75E.06 | 23.228112 | Dn | adiponectin precursor |
| RETN | 0.000591 | 0.023702 | 5.8983441 | Up | resistin |
| PLA2G5 | 6.94E-06 | 0.00066 | 4.88 | Dn | phospholipase A2, group V |
| PLA2G4A | 0.007923 | 0.15 | 2.2891944 | Dn | phospholipase A2, group IVA (cytosolic, calcium-dependent) |
| FFAR2 | 3.12E-05 | 0.002298 | 20.218163 | Up | free acid receptor 2 free fatty acid receptor |
| AGT | 3.08E-05 | 0.002286 | 31.794603 | Dn | angiotensinogen (serpin peptidase inhibitor, clade A, member 8) |
| NFKBIA | 2.95E-06 | 0.000328 | 4.2463992 | Up | nuclear factor of kappa light polypeptide gene enhancer in B-cells inhibitor, alpha |
| TLR4 | 0.001656 | 0.05 | 2.6760777 | Up | toll-like receptor 4 precursor |
| FOS | 1.14E-11 | 5.61E-09 | 9.9343116 | Up | PBJ murine osteosarcoma viral oncogene homolog |
| JUNB | 8.84E-09 | 2.49E-06 | 6.0709404 | Up | jun B prolo-oncogene |
| AQP9 | 0.001899 | 0.058 | ' 53.713767 | Up | aquaporin 9 |
| SOAT1 | 0.008319 | 0.156048 | 2.9381132 | Up | sterol O-acyltransferase 1 |
| SMAD6 | 0.001305 | 0.043867 | 2.9369323 | Up | SMAD family member 6 |
| EDNRB | 8.66E-06 | 0.000796 | 3.6630871 | Up | endothelin B receptor precursor |
| NKX2-5 | 0.002877 | 0.0777 | 2.3729636 | Dn | homeobox protein Nkx-2.5 |
| GATA4_ CANFA | 0.005836 | 0.125586 | 2.2325771 | Dn | Transcription factor GATA-4 |
| PTGS2 (COX-2) | 1.79E-08 | 448E-06 | 12.511215 | Up | prostaglandin-endoperoxide synthase 2 (prostaglandin G/H synthase and cyclooxygenase) |
| IL8_CANFA | 0 | 0 | 135.16333 | Up | Interleukin-8 |
| IL6 | 1-44E-10 | 5.68E-08 | 584.83669 | Up | interleukin-6 precursor |
| MYH1_CANFA | 0.000126 | 0.007023 | 4 | Dn | myosin, heavy chain 1, skeletal muscle, adult [Source:HGNC Symbol;Acc:7567] |

The top 15 left ventricle gene expression markers differentially expressed (Table 2) were selected from the Table 1 for real time quantitative reverse transcription PCR assay (qPCR). The results showed 12 genes with p values of less than 0.05 (p<0.05), while 14 with p values of less than 0.1 (p<0.10). In addition, 14 genes showed consistency in the of gene expressional changes with RNA-seq.

**Table 2**

| **Gene name** | **P value** | **Fold Change** | **Description** |
|---|---|---|---|
| ACSL1 | 0.0015 | -2.17 | acyl-CoA synthetase long-chain family member 1 |
| COL14A1 | 0.015 | -1.94 | collagen, type XIV, alpha 1 |
| FADS1 | 0.1224 | -2.11 | fatty acid desastruase 1 |
| HOPX | 0 | 3.15 | HOP homeobox |
| LIPE | 0.0133 | -1.79 | lipase, hormone-sensitive |
| MLYCD | 0.0042 | -1.75 | malonyl-CoA decarboxylase |
| MMP15 | 7.00E-04 | -1.73 | matrix metallopeptidase 15 |
| MMP8 | 0 | 155.13 | matrix metallopeptidase 8 |
| MMP9 | 0.0041 | 256.22 | matrix metalloproteinase-9 precursor |
| NOS2 | 0.0061 | -11 | Nitric Oxide Synthase, inducible |
| NOS3 | 0 | 6.4 | Nitric Oxide Synthase, endothelial |
| NPPA | 0.0538 | 6.89 | Natriuretic peptides A Atrial natriuretic factor |
| Serpine 1 (PAI-1) | 0 | 43.16 | SERPINE1 serpin peptidase inhibitor, clade E |
| TGFB3 | 0.091 | -1.35 | transforming growth factor, beta 3 |
| TIMP1 | 0 | 51.77 | metalloproteinase inhibitor 1 precursor |

The top 20 mitral valve gene expression markers differentially expressed in mitral valve tissue are identified in Table 3.

**Table 3**

| **Gene name** | **P value** | **FDR** | **Fold change** | **Up/Rn** | **Description** |
|---|---|---|---|---|---|
| NOS3 | 9.74E-05 | 0.00627 | 4.62 | Up | Nitric Oxide Synthase, endothelial |
| COL6A5 (COL29A1) | 6.03E-08 | 0.000127 | 9 | Up | collagen, type VI, alpha 5 |
| Serpinel (PA1-1) | 1.28E-05 | 0.007573 | 5.2 | Up | SERPINE1 serin peptidase inhibitor, clade E (nexin, plasminogen activator inhibitor type 1). member 1 |
| SELP | 3.49E-09 | 8.63E-06 | 12.74 | Up | selectin **P** (granule membrane protein 140kDa, antigen CD62) |
| ACSL1 | 0.004 | 0.33 | 2.57 | Dn | acyl-CoA synthetase long-chain family member 1 |
| AD1POQ | 0.000163 | 0.043 | 4.799 | Dn | actiponectin precursor |
| O3FAR1 | 0.008104 | 0.51726 | 9.273 | Dn | omega- 3 fatty acid receptor 1 |
| FABP4 | 0.0031 | 0.27911 | 2.913 | Dn | homolog to human fatty acid binding protein 4, adipocyte [Source:HGNC Symbol;Acc:3559] |
| SLC27A6 | 0.007399 | 0.48063 | 4.013 | Up | solute carrier family 27 (fatty acid transporter). member 6 |
| EDN1_CANFA | 0.003495 | 029919 | 3.385 | Up | Endothelin-1 |
| CD74 | 0.000923 | 0.12893 | 4.251 | Up | CD74 molecule, major histocompatibility complex, class II invariant chain |
| GSTP1 | 0.004974 | 0.38165 | 2.563 | Dn | glutathione S-transferase pi 1 |
| MOST1 | 0.001648 | 0.19365 | 3.264 | Dn | microsomal glutathione S-transferase 1 |
| MYC | 0.004623 | 0.36039 | 2.667 | Up | myc proto-oncogene protein |
| MT2 CANFA | 2.44E-05 | 0.01121 | 7.26 | Up | Metaliothionein-2 |
| IL8 CANFA | 1.01E-05 | 0.00621 | 18.31 | Up | Interleukin-8 |
| IL6 | 0.000729 | 0.11483 | 14.89 | Up | interleukin-6 precursor |
| PLIN1 | 0.000319 9 | 0.06222 | 4.391 | Dn | perilipin 1 |
| CLDN1 | 7.12E-06 | 0.00575 | 6.13 | Up | claudin 1 |
| AGT | 5.53-06 | 0.00512 | 8.07 | Dn | AGT angiotensionogen (serpin peptidase isthibitor, clade A, member 8) |

The top 5 left mitral valve gene expression markers expressed and shown in Table 4 were selected from the Table 2 for qPCR assay. The results showed 2 genes with p values of less all five genes showed consistency in the direction of gene expressional changes with RNA-seq.

**Table 4**

| **Gene name** | **P value** | **Fold Change** | **Description** |
|---|---|---|---|
| NOS3 | 0.0399 | 3.31 | Nitric Oxide Synthase, endothelial |
| COL6A5 (COL29A1) | 0.2364 | 7.9 | collagen, type VI, alpha 5 |
| Serpinel (PAI-1) | 0.2853 | 5.23 | SERPINE 1 serpin peptidase inhibitor, clade E |
| SELF | 0.102 | 17.02 | selectin P |
| ACSL1 | 0.0248 | -2.59 | acyl-CoA synthetase long-chain family member 1 |

The 812 differentially expressed genes in the left ventricle tissue are identified in Table 5.

**Table 5**

| **Gene name** | **P value** | **FDR** | **Fold change** | **Up/Dn** | **Description** |
|---|---|---|---|---|---|
| CA3 | 0.000828 | 0.03087 | 9999 | Dn | carbonic anhydrase 3 |
| CRHR1 | 0.000372 | 0.016316 | 9999 | Dn | corticotropin releasing hormone receptor 1 |
| TUSC5 | 0.007243 | 0.142657 | 9999 | Dn | rumor suppressor candidate 5 |
| Xist_exon4 | 0.003242 | 0.083712 | 9999 | Dn | X-chromosome inactivation gene exon 4 |
| novel gene | 3.06E-05 | 0.002281 | 9999 | Dn | Uncharacterized protein |
| MYH13 | 69.5E-12 | 3.55E-09 | 60.39 | Dn | myosin, heavy chain 13, skeletal muscle |
| PCP2 | 4.76E-10 | L82E-07 | 48.44 | Dn | Purkinje cell protein 2 |
| MYH4_CANPA | 0.000981 | 1 | 48.34 | Dn | Myosin-4 |
| VWCE | 4.42E-08 | 9.99E-06 | 44.28 | Dn | von Willebrand factor C and EGF domains |
| AGT | 3.08E-05 | 0.002286 | 31.79 | Dn | angiotensinogen (serpin peptidase inhibitor, clade A, member 8) |
| MKRN2-AS1 | 0.005829 | 0.125586 | 23.52 | Dn | MKRN2 antisense RNA 1 |
| ADIPOQ | 5.96E-09 | 1.75E-06 | 23.23 | Dn | adiponectin precursor |
| PLIN1 | 1.77E-08 | 4.48E-06 | 22.27 | Dn | perilipin 1 |
| NOS2 | 0.000223 | 0.0114 | 21.84 | Dn | nitric oxide synthase, inducible |
| CCDC85C | 3.25E-09 | 1.07E-06 | 21.24 | Dn | coiled-coil domain containing 85C |
| | FOXR1 4,33E-05 | 0,003072 | 19.56 | Dn | forkhead box R1 |
| DPP10 | 0.000332 | | 18.13 | Dn | dipeptidyl-peptidase 10 (non-functional) |
| novel gene | 0.001187 | 0.040927 | 17.68 | Dn | Uncharacterized protein |
| GLT25D2 | 0.001979 | 0.059175 | 17.35 | Dn | glycosyltransferase 25 domain containing 2 |
| novel gene | 0.000254 | 0.012429 | 16.91 | Dn | Uncharacterized protein |
| XNF835 | 0.001743 | 0.054143 | 16.8 | Dn | zinc finger protein 835 |
| SDSL | 0.000274 | 0.013077 | 15.18 | Dn | serine dehydratase-like |
| RCOR2 | 7.20E-07 | 0.000106 | 14.32 | Dn | REST corepressor 2 |
| CYP1A1 | 1 .81E-07 | 3.29E-05 | 13.74 | Dn | cytochrome P450, family 1, subfamily A, polypeptide 1 |
| novel gene | 0.000912 | 0.033432 | 12.31 | Dn | Uncharacterized protein |
| NDST3 | 0.000748 | 1 | 12.29 | Dn | N-deacetylase/N-sulfotransferase (heparan glucosaminyl) 3 |
| novel gene | 0.002955 | 0.078949 | 12.11 | Dn | Uncharacterized protein |
| TMOD4 | 0.003582 | 0.089802 | 11.61 | Dn | tropomodulin 4 (muscle) |
| FISX83_CANFA | 0.000122 | 0.006896 | 11.29 | Dn | plasma kallikrein |
| novel gene | 2.20E-06 | 0.00026 | 10.77 | Dn | Uncharacterized protein |
| novel gene | 7.33E-07 | 0.000106 | 10.71 | Dn | Uncharacterized protein |
| Q3HTT6_CANFA | 0.000426 | 0.018089 | 10.62 | Dn | Hydroxysteroid 1I -beta dehydrogenase 2 |
| TCEAL7 | 0.000124 | 0.006995 | 10.56 | Dn | transcription elongation factor A (SII)-like 7 |
| PPP1R16B | 4.51E-09 | 1.38E-06 | 10.36 | Dn | protein phosphatase 1, regulatory subunit 16B |
| CD300LG | 4.35E-05 | 0.003072 | 10.19 | Dn | CD300 molecule-like family member g |
| NGFR | 1.02E-06 | 0.000139 | 9.89 | Dn | nerve growth factor receptor |
| novel gene | 5.44E.07 | 8.52E-05 | 9.47 | Dn | Uncharacterized protein |
| STAPI | 0.009371 | 0.167166 | 9.36 | Dn | signal transducing adaptor family member 1 |
| CCL24_CANFA | 0.007727 | 0.149102 | 9.34 | Dn | C-C motif chemokine 24 |
| CAPNI 1 | 0.000524 | 0.021472 | 9.3 | Dn | calpain I1 |
| APLNR | 1.74E-06 | 0.000213 | 9.21 | Dn | apelin receptor |
| novel gene | 8.42E-08 | 1.68E-05 | 9.12 | Dn | Uncharacterized protein |
| FAM166B | 0.001214 | 0.041655 | 9.08 | Dn | family with sequence similarity 166, member B |
| CAMKV | 0.00425 | 0.102081 | 8.98 | Dn | CaM kinase-like vesicle-associated |
| novel gene | 1.06E-06 | 0.000143 | 8.8 | Dn | Uncharacterized protein |
| DAO | 0.003994 | 0.097641 | 8.37 | Dn | D-amino-acid oxidase |
| novel gene | 4.96E-05 | 0.003355 | 8.19 | Dn | Uncharacterized protein |
| SYT7 | 0.000373 | 0.016316 | 8.15 | Dn | synaptotagmin VII |
| PCDH12 | 2.02E-08 | 4.97E-06 | 7.97 | Dn | protocadherin 12 |
| CMA1_CANFA | 0.003027 | 0.0801 | 7.93 | Dn | Chymase |
| LRRC14B | 4.15E-07 | 6.72E-05 | 7.9 | Dn | leucine rich repeat containing 14B |
| novel gene | 0.004837 | 0.11061 | 7.76 | Dn | Uncharacterized protein |
| EXPH5 | 2.27E-08 | 5.48E-06 | 7.61 | Dn | exophilin 5 |
| BMF | 1.19E-05 | 0.001003 | 7.47 | Dn | Bcl2 modifying factor |
| RASD2 | 0.002273 | 0.06597 | 7.37 | Dn | RASD family, member 2 |
| CDH15 | 4.24E-05 | 0.003032 | 7.32 | Dn | cadherin 15, type 1, M-cadherin (myotubule) |
| CIDEC | 0.000362 | 0.016161 | 7.25 | Dn | cell death-inducing DFFA-like effector c |
| ANGPTL1 | 0.001494 | 0.04882 | 7.2 | Dn | angiopoietin-related protein 1 precursor |
| DDN | 9.92E-06 | 0.00086 | 7.03 | Dn | dendrin |
| WNT5A | 2.41E-06 | 0.000277 | 6.96 | Dn | Protein Wnt |
| NIPAL1 | 8.89E-06 | 0.000806 | 6.74 | Dn | NIPA-like domain containing 1 |
| novel gene | 7.44E-05 | 0.004704 | 6.71 | Dn | Uncharacterized protein |
| novel gene | 6.44E-06 | 0.000634 | 6.56 | Dn | Uncharacterized protein |
| TTYH2 | 125E-06 | 0.000161 | 6.55 | Dn | tweety homolog 2 (Drosophila) |
| Q9GK59_CANFA | 1.70E-06 | 0.00021 | 6.5 | Dn | Na+/H+ exchanger isoform 1 |
| AADAC | 2.54E-06 | 0.000287 | 6.45 | Dn | arylacetamide deacetylase |
| novel gene | 0.001232 | 0.042037 | 6.32 | Dn | Uncharacterized protein |
| PPP1R1B | 0.000688 | 0.027042 | 6.23 | Dn | protein phosphatase 1, regulatory (inhibitor) subunit 1B |
| D91Q20_CANFA | 2.65E-06 | 0.000297 | 6.2 | Dn | stromelysin-3 precursor |
| novel gene | 0.007787 | 0.149537 | 6.02 | Dn | Uncharacterized protein |
| GFRA2 | 0.008919 | 1 | 5.99 | Dn | GDNF family receptor alpha 2 |
| SLITRK6 | 9.38E-05 | 0.005749 | 5.99 | Dn | SLIT and MTRK-like family, member 6 |
| LRRC38 | 7.02E-05 | 0.004546 | 5.94 | Dn | leucine rich repeat containing 38 |
| SORCS1 | 9.23E-06 | 0.000811 | 5.92 | Dn | sortilin-related VPS10 domain containing receptor 1 |
| novel gene | 0.000796 | 0.029983 | 5.86 | Dn | Uncharacterized protein |
| CPA3 | 0.000125 | 0.007023 | 5.86 | Dn | carboxypeptidase A3 (mast cell) |
| NRIP2 | 0.000368 | 0.016316 | 5.81 | Dn | nuclear receptor interacting protein 2 |
| CCDC8 | 5.85E-05 | 0.003879 | 5.76 | Dn | coiled-coil domain containing 8 |
| TDRD1 | 2.70E-05 | 0.002054 | 5.74 | Dn | tudor domain containing 1 |
| ITPKB | 1.11E-06 | 0.000147 | 5.69 | Dn | inostol-trisphosphate 3-kinase B |
| FMO2 | 8.14E-06 | 0.000759 | 5.61 | Dn | flavin containing monooxygenase 2 (non-functional) |
| ART5 | 0.002546 | 0.07121 | 5.58 | Dn | ADP-ribosyltransferase 5 |
| ACOT1 | 8.63E-05 | 0.005357 | 5.58 | Dn | acyl-CoA thioesterase 1 |
| SV2 B | 0.007766 | 0.149341 | 5.54 | Dn | synaptic vesicle glycoprotein 2B |
| IGSF11 | 0.001745 | 0.054143 | 5.49 | Dn | immunoglobulin superfamily, member 11 |
| ABCC8 | 0.000271 | 0.012961 | 5.49 | Dn | ATP-binding cassette, sub-family C (CFTR/MRP), member 8 |
| novel gene | 0.004415 | 0.104947 | 5.4 | Dn | Uncharacterized protein |
| FZD8 | 9.21E-06 | 0.000811 | 5.38 | Dn | frizzled family receptor 8 |
| OLFML2A | 5.00E-06 | 0.0005 18 | 5.36 | Dn | olfactomedin-like 2A |
| NEURL1B | 0.007574 | 0.147283 | 5.35 | Dn | neuralized homolog 1B (Drosophila) |
| IFIT2 | 0.000202 | 0.010529 | 5.34 | Dn | interferon-induced protein with tetratricopeptide repeats 2 |
| PROM1 | 0.000555 | 0.022627 | 5.3 | Dn | prominin 1 |
| CA8 | 0.008022 | 0.152347 | 5.3 | Dn | carbonic anhydrase VIII |
| ZNF446 | 0.002775 | 0.076218 | 5.27 | Dn | zinc finger protein 446 |
| FREM2 | 3.37E-05 | 0.002461 | 5.23 | Dn | FRAS1 related extracellular matrix protein 2 |
| TMC5 | 0.000778 | 0.029489 | 5.23 | Dn | transmembrane channel-like 5 |
| ZBTB20 | 0.008848 | 0.162645 | 5.21 | Dn | zinc finger and BTB domain containing 20 |
| novel gene | 0.00086 | 0.03197 | 5.1 6 | Dn | Uncharacterized protein |
| INHA | 4.23E-06 | 0.00045 | 5.14 | Dn | inhibin, alpha |
| GDA | 0.0094 | 0.167457 | 5.12 | Dn | guanine deaminase |
| novel gene | 0.00189 | 1 | 5.07 | Dn | Uncharacterized protein |
| RARG | 0.000165 | 0.008966 | 5.03 | Dn | retinoic acid receptor, gamma |
| PLXDC1 | 1.15E-06 | 0.000151 | 5.03 | Dn | plexin domain containing 1 |
| AGXT2L1 | 0.002847 | 0.077432 | 5.02 | Dn | alanine-glyoxylate aminotransferase 2-like 1 |
| novel gene | 0.000283 | 0.01338 | 4.99 | Dn | Uncharacterized protein |
| LGR6 | 0.009539 | 1 | 4.98 | Dn | leucine-rich repeat containing G protein-coupled receptor 6 |
| FAM212A | 0.000748 | 0.028562 | 4.93 | Dn | family with sequence similarity 212, member A |
| PLA2G5 | 6.94E-06 | 0.00066 | 4.89 | Dn | phospholipase A2, group V |
| PPP1R1C | 0,000261 | 0.012607 | 4.87 | Dn | protein phosphatase 1, regulatory (inhibitor) subunit 1C |
| LAMA3 | 0.000731 | 1 | 4.79 | Dn | laminin, alpha 3 |
| TCF15 | 6.47E-06 | 0.000634 | 4.72 | Dn | transcription factor 15 (basic helix-loop-helix) |
| KCNK13 | 1.07E-05 | 0.000919 | 4.67 | Dn | potassium channel, subfamily K, member 13 |
| SEMA3G | 9.07E-05 | 0.005611 | 4.67 | Dn | sema domain, immunoglobulin domain (Ig), short basic domain, secreted, (semaphorin) 3G |
| GALNTL1 | 0.00148 | 0.048469 | 4.67 | Dn | UDP-N-acetyl-alpha-D-galactosamine:polypepide N-acetylgalactosaminyltransferase-like 1 |
| ADCYAPIRI | 0.000106 | 0.0063 | 4.65 | Dn | adenylate cyclase activating polypeptide 1 (pituitary) receptor type 1 |
| GBP6 | 0.000328 | 0.015102 | 4.63 | Dn | guanylate binding protein family, member 6 |
| CSAD | 0,001791 | 0.055248 | 4.59 | Dn | cysteine salfinic acid decarboxylase |
| SPTBN2 | 1.28E-05 | 0.001071 | 4.58 | Dn | spectrin, beta, non-erythrocytic 2 |
| ATXN7L1 | 0.003493 | 0.08836 | 4.58 | Dn | ataxin 7-like 1 |
| CCDC68 | 0.001016 | 0.036389 | 4.55 | Dn | coiled-coil domain containing 68 |
| GPD1 | 6.15E-07 | 9.42E-05 | 4.53 | Dn | glycerol-3-phosphate dehydrogenase 1 (soluble) |
| HMGCLL1 | 0.003106 | 0.081562 | 4.53 | Dn | 3-hydroxymethyl-3-methylglutaryl-CoA lyase-like 1 |
| GR1A3 | 0.004614 | 1 | 4.49 | Dn | glutamate receptor, ionotropic, AMPA 3 |
| FBLN7 | 0.003145 | 0.082416 | 4.48 | Dn | fibulin 7 |
| NOX5 | 0.0001 16 | 0.006675 | 4.48 | Dn | NADPH oxidase 5 |
| B2CRU9_CANFA | 0.004014 | 0.097943 | 4.46 | Dn | P2Y purinoceptor 2 |
| MAL | 0.005642 | 0.123277 | 4.44 | Dn | myelin and lymphocyte protein |
| NAALAD2 | 0.000235 | 0.011867 | 4.42 | Dn | N-acetylated alpha-linked acidic dipeptidase 2 |
| novel gene | 0.000733 | 0.028226 | 4.41 | Dn | Uncharacterized protein |
| OSR2 | 0.004473 | 0.105793 | 4.39 | Dn | odd-skipped related 2 (Drosophila) |
| novel gene | 0.005667 | 0.123441 | 4.39 | Dn | Uncharacterized protein |
| TRYT_CANFA | 0.00293 | 0.078586 | 4.37 | Dn | Tryptase |
| FAM198B | 6.73E-07 | 9.98E-05 | 4.36 | Dn | family with sequence similarity 198, member B |
| SDC1 | 0.000745 | 0.028562 | 4.35 | Dn | syndecan 1 |
| LAMC3 | 0.000128 | 0.007102 | 4.33 | Dn | laminin, gamma 3 |
| PGBD5 | 0.002066 | 0.061044 | 4.32 | Dn | piggyBac transposable element derived 5 |
| RAB33A | 00007 | 0.027381 | 4.3 | Dn | RAB33A, member RAS oncogene family |
| FNDC1 | 0.00377 | 1 | 4.29 | Dn | fibronectin type HI domain containing 1 |
| MART | 5.05E-05 | 0.00338 | 4.28 | Dn | microtubule-associated protein tau |
| novel gene | 0.009175 | 0. 1 66456 | 4.27 | Dn | Uncharacterized protein |
| ANGEL1 | 0.002127 | 0.062132 | 4.22 | Dn | angel homolog 1 (Drosophila) |
| NPTXR | 0.000445 | 0.018801 | 4.21 | Dn | neuronal pentraxin receptor |
| BCAR3 | 0,000173 | 0.009294 | 4.21 | Dn | breast cancer anti-estrogen resistance 3 |
| Q4PLA8_CANFA | 0.009116 | 1 | 4.18 | Dn | multidrug resistance protein 1 |
| SLC2A5 | 0.003189 | 0.082815 | 4.16 | Dn | solute carrier family 2 (facilitated glucose/fructose transporter), member 5 |
| MYH1_CANFA | 0.000126 | 0.007023 | 4.13 | Dn | Myosin-1 |
| SLC8A3 | 0.002516 | 0.070652 | 4.13 | Dn | solute carrier family 8 (sodium/calcium exchanger), member 3 |
| FBN2 | 4.24E-06 | 0.00045 | 4.13 | Dn | fibrillin 2 |
| ADAMTS7 | 3.49E-06 | 0.000378 | 4.12 | Dn | ADAM metallopeptidase with thrombospondin type 1 motif, 7 |
| NTHL1 | 0.003728 | 0.092452 | 4.12 | Dn | nth endonuclease III-like 1 (E. coli) |
| EXTL1 | 6.74E-05 | 0.004384 | 4.11 | Dn | exostoxes (multiple-)-like 1 |
| PLP1 | 0.000446 | 0.018818 | 4.09 | Dn | proteolipid protein 1 |
| PLSCR4 | 4.88E-05 | 0.003331 | 4.07 | Dn | phospholipid scramblase 4 |
| SYNPQ2L | 5.88E-06 | 0.000592 | 4.07 | Dn | synaptopodin 2-like |
| TNFSF10 | 5.43E-06 | 0.000559 | 4.06 | Dn | tumor necrosis factor (ligand) superfamily, member 10 |
| N4BP3 | 4.91E-05 | 0.003337 | 4.05 | Dn | NEDD4 binding protein 3 |
| GAB3 | 0.005975 | 0.127334 | 4.04 | Dn | GRB2-associated binding protein 3 |
| SOX12 | 0.006405 | 0.133206 | 4.04 | Dn | SRY (sex determining region Y)-box 12 |
| FAM110B | 0.001839 | 0.056597 | 4.03 | Dn | family with sequence similarity 110, member B |
| novel gene | 0.000175 | 0.009368 | 4.01 | Dn | Uncharacterized protein |
| NLRX1 | 3.14E-05 | 0.002303 | 3,94 | Dn | NLR family member X1 |
| TMEM164 | 8.73E-06 | 0.000798 | 3.91 | Dn | transmembrane protein 164 |
| CSGALNACT1 | 0.000172 | 0.009272 | 3.9 | Dn | chondroitin sulfate N-acetylgalactosaminyltransferase 1 |
| FAM26F | 0.004877 | 0.11079 | 3.9 | Dn | family with sequence similarity 26, member F |
| GJA1 | 9.08E-06 | 0.000811 | 3.9 | Dn | gap junction alpha-1 protein |
| MOGAT1 | 2.48E-05 | 0.001902 | 3.89 | Dn | monoacylglycerol O-acyltransferase 1 |
| KANK4 | 0.00859 | 1 | 3.88 | Dn | KN motif and ankyrin repeat domains 4 |
| KIF26A | 0.006293 | 0.132067 | 3.85 | Dn | kinesin family member 26A |
| TTC34 | 4.80E-05 | 0.00331 | 3.82 | Dn | tetratricopeptide repeat domain 34 |
| SLC12A7 | 0.000146 | 0.008038 | 3.82 | Dn | solute carrier family 12 (potassium/chloride transporters), member 7 |
| TK1 | 0.00071 | 0.027567 | 3.81 | Dn | thymidine kinase 1, soluble |
| novel gene | 0.000107 | 0.0063 | 3.81 | Dn | Uncharacterized protein |
| A5H028_CANFA | 3.77E-05 | 0.002735 | 3.81 | Dn | 2-5A-dependent ribonuclease |
| VIPR1 | 0.009159 | 0.166456 | 3.76 | Dn | vasoactive intestinal peptide receptor 1 |
| PER3 | 0.000103 | 0.006229 | 3.75 | Dn | period homolog 3 (Drosophila) |
| PLXNB1 | 1.72E-05 | 0.001393 | 3.74 | Dn | plexin B1 |
| ZBTB40 | 0.000124 | 0.006995 | 3.73 | Dn | zinc finger and BTB domain containing 40 |
| novel gene | 0.00029 | 0.01362 | 3.73 | Dn | Uncharacterized protein |
| FMO3 _CANFA | 0.00762 | 0.147774 | 3.73 | Un | Dimethylaniline monooxygenase 3 |
| DACT1 | 0.009206 | 0.166582 | 3.69 | Dn | dapper, antagonist of beta-catenin, homolog 1 (Xenopus laevis) |
| CYB561 | 0.000286 | 0.013436 | 3.69 | Dn | cytochrome b-561 |
| ACPP | 0.008432 | 0.15697 | 3.67 | Dn | acid phosphatase, prostate |
| PCHO1 | 0.005887 | 0.126031 | 3.65 | Dn | FCH domain only 1 |
| C1orf192 | 0.000379 | 0,016556 | 3.64 | Dn | chromosome 1 open reading frame 192 |
| C19orf68 | 0.00975 | 0.170771 | 3.63 | Dn | chromosome 19 open reading frame 68 |
| novel gene | 0.001972 | 0.059114 | 3.57 | Dn | Uncharacterized protein |
| SEMA5B | 0.000974 | 0.034962 | 3.57 | Dn | sema domain, seven thrombospondin (type 1 and type 1-like), repeats (type 1 type transmembrane domain (TM) and short cytoplasmic domain, (semaphorin) 5B |
| ABCA3 | 0.00125 | 0.04245 | 3.56 | Dn | ATP-binding cassette, sub-family A (ABCI), ), member 3 |
| FGFBP1 | 0.000312 | 0.014502 | 3.54 | Dn | fibroblast growth factor binding protein 1 |
| SPOCK2 | 0.003341 | 0.085787 | 3.53 | Dn | sparc/osteonectin, cwcy and kazal-like domains proteoglycan (testican) 2 |
| TMEM97 | 0.000414 | 0.017753 | 3.52 | Dn | transmembrane protein 97 |
| Q3HTT9_CANFA | 0.00244 | 0.069093 | 3.51 | Dn | Mineralocorticoid receptor |
| WISP2 | 0.000106 | 0.0063 | 3.47 | Dn | WNT1 inducible signaling pathway protein 2 |
| ASB18 | 0.000897 | 0.03297 | 3.46 | Dn | ankyrin repeat and SOCS box containing 18 |
| AFAPIL2 | 0.001747 | 0.054143 | 3.45 | Dn | actin filament associated protein 1-like 2 |
| GLTPD1 | 0.000583 | 0.023517 | 3.43 | Dn | glycolipid transfer protein domain containing 1 |
| EREB3 | 0.007481 | 0.145693 | 3.41 | Dn | v-erb-b2 erythroblastic leukemia viral oncogene homolog 3 (avian) |
| PHACTR3 | 0.001326 | 0.044373 | 3.41 | Dn | phosphatase and actin regulator 3 |
| SLC4A5 | 0.003419 | 0.087146 | 3.4 | Dn | solute carrier family 4, sodium bicarbonate cotransporter, member 5 |
| MMP15 | 0.00016 | 0.008734 | 3.39 | Dn | matrix metallopeptidase 15 (membrane-inserted) |
| CIQTNF2 | 0.009886 | 0.17211 | 3.39 | Dn | C1q and tumor necrosis factor related protein 2 |
| NAV3 | 0.002386 | 0.067834 | 3.39 | Dn | neuron navigator 3 |
| FAT1 | 0.000195 | 0.010238 | 3.38 | Dn | FAT tumor suppressor homolog 1 (Drosophila) |
| CLDN4 | 0.003857 | 0.09497 | 3.38 | Dn | claudin 4 |
| ATP2B4 | 0.000216 | 0.011068 | 3.37 | Dn | ATPase, Ca++ transporting, plasma membrane 4 |
| INPP5J | 0.002564 | 0.071422 | 3.35 | Dn | inositol polyphosphate-5-phosphatase J |
| NT5C1 A | 0.00037 | 0.016316 | 3.34 | Dn | cytosolic IA |
| GLE1 | 0.004859 | 0.110726 | 3.3 3 | Dn | GLE1 RNA export mediator homolog (yeast) |
| FAM180B | 0.006588 | 0.134969 | 3.32 | Dn | family with sequence similarity 180, member B |
| AMOT | 0.009176 | 1 | 3.32 | Dn | angiomotin |
| SCN2B | 0.002901 | 0.077982 | 3.31 | Dn | sodium channel subunit beta-2 |
| CLEC3B | 7.64E-05 | 0.004809 | 3.31 | Dn | C-type lectin domain family 3, member B |
| novel gene | 0.003436 | 0.087413 | 3.31 | Dn | Uncharacterized protein |
| C12orf52 | 0.00471 1 | 0.109717 | 3.3 | Dn | chromosome 12 open reading frame 52 |
| THSD1 | 0.002507 | 0.070544 | 3.29 | Dn | thrombospondin, type I, domain containing 1 |
| SPESP1 | 0.009335 | 0.1 67087 | 3.29 | Dn | sperm equatorial segment protein 1 |
| ZBTB12 | 0.00976 | 0.170771 | 3.26 | Dn | zinc finger and BTB domain containing 12 |
| OBSCN | 0.004451 | 0.105623 | 3.26 | Dn | obscurin, cytoskeletal calmodulin and titin-interacting RhoGEP |
| GPT2 | 0.003692 | 0.091729 | 3.23 | Dn | glutamic pyruvate transaminase (alanine aminotransferase) 2 |
| TTC28 | 0.006094 | 0.129258 | 3.22 | Dn | tetratricopeptide repeat domain 28 |
| Q8HYR4_CANFA | 0.006012 | 0.127722 | 3.22 | Dn | multidrug resistance-associated protein 1 |
| P2RY1 | 0.001439 | 0.04734 | 3.21 | Dn | P2Y purinoceptor 1 |
| WSCD1 | 0.005297 | 0.117807 | 321 | Dn | WSC domain containing 1 |
| CIDEA | 0.002953 | 0.078949 | 3.21 | Dn | cell death-inducing DFFA-like effector a |
| SEMA6C | 0.001942 | 0.058326 | 3.21 | Dn | sema domain, transmembrane domain (TM), and cytoplasmic domain, (semaphorin) 6C |
| OLFML1 | 0.001519 | 0.049523 | 3.2 | Dn | olfactomedin-like 1 |
| novel gene | 0.000939 | 0.034061 | 3.19 | Dn | Uncharacterized protein |
| PABPC1L | 0.001283 | 0.043266 | 3.19 | Dn | poly(A) binding protein, cytoplasmic 1-like |
| FLYWCH2 | 0.008363 | 0.156249 | 3.18 | Dn | FLYWCH family member 2 |
| ITGA11 | 0.00249 | 0.070205 | 3.18 | Dn | integrin, alpha 11 |
| IFI35 | 0008925 | 0.163633 | 3.17 | Dn | interferon-induced protein 35 |
| MPP2 | 0003447 | 0.087529 | 3.15 | Dn | membrane protein, palmitoylated 2 (MAGUK p55 subfamily member 2) |
| FAM78A | 0.000385 | 0.01675 | 3.14 | Dn | family with sequence similarity 78, member A |
| MYH7B | 0.0005319 | 0.11809 | 3.13 | Dn | myosin, heavy chain 7B, cardiac muscle, beta |
| TSPAN9 | 7.85E-05 | 0.004898 | 3.12 | Dn | letraspanin 9 |
| novel gene | 0.001121 | 0.039244 | 3.12 | Dn | Uncharacterized protein |
| NFA Q5SBJ3_CANFA | 0.004272 | 0.102081 | 3.11 | Dn | Excision repair cross-complementing 2 |
| C5orf65 | 0.0004 1 | 0.017649 | 3.08 | Dn | chromosome 5 open reading frame 65 |
| GPR162 | 0.00011 2 | 0.006503 | 3.08 | Dn | G protein-coupled receptor 162 |
| VPS13D | 0.001787 | 0.055248 | 3.07 | Dn | vacuolar protein sorting 13 homolog D (S. cerevisiae) |
| ST3GAL2 | 0.008279 | 0.155575 | 3.06 | Dn | ST3 beta-galactoside alpha-2,3-sialyltransferase 2 |
| ASPA | 0.002052 | 0.060991 | 3.06 | Dn | aspartoacylase |
| novel gene | 0.001746 | 0.054143 | 3.05 | Dn | Uncharacterized protein |
| Q5YLN6_CANFA | 0.002338 | 0.067301 | 3.03 | Dn | sodium channel protein type 5 subunit alpha |
| KIAA1161 | 0.001279 | 0.043222 | 3.03 | Dn | KIAA1161 |
| FYCO1 | 0.000655 | 0.026009 | 3 | Dn | FYVE and coiled-coil domain containing 1 |
| ANKRD29 | 0.001404 | 0.046324 | 2.99 | Dn | ankyrin repeat domain 29 |
| SLC25A42 | 0.001858 | 0.056696 | 2.99 | Dn | solute carrier family 25, member 42 |
| ANK1 | 0.000351 | 0.01596 | 2.99 | Dn | ankyrin 1, erythrocytic |
| ASB13 | 0.001616 | 0.051927 | 2.98 | Dn | ankyrin repeat and SOCS box containing 13 |
| ACSL1 | 0.001397 | 0.04619 | 2.98 | Dn | acyl-CoA synthetase long-chain family member 1 |
| RSG1 | 0.007732 | 0.149102 | 2.98 | Dn | REM2 and RAB-like small GTPase 1 |
| X1RP2 | 0.002868 | 0.0777 | 2.95 | Dn | xin actin-binding repeat containing 2 |
| C1QTNF7 | 0.005589 | 0.122318 | 2.94 | Dn | Clq and tumor necrosis factor related protein 7 |
| MOCS 1 | 0.003381 | 0.086517 | 2.93 | Dn | molybdenum cofactor synthesis 1 |
| PLCD1 | 0.005556 | 0.121781 | 2.93 | Dn | phospholipase C, delta 1 |
| SETD7 | 0.004031 | 0.098034 | 2.92 | Dn | SET domain containing (lysine methyltransferase) 7 |
| ABCA2 | 0.002029 | 0.060418 | 2.9 | Dn | ATP-binding cassette, sub-family A (ABC1), member 2 |
| FADS1 | 0.002074 | 0.061091 | 2.87 | Dn | fatty acid desaturase 1 |
| novel gene | 0.00093 | 0.033826 | 2.86 | Dn | Uncharacterized protein |
| SARDH | 0.000518 | 0.021373 | 2.86 | Dn | sarcosine dehydrogenase |
| LGALS9 | 0.006433 | 0.133387 | 2.85 | Dn | galectin-9 |
| EYA1 | 0.000415 | 0.017753 | 2.85 | Dn | eyes absent homolog 1 (Drosophila) |
| NFIX | 0.002725 | 0.075148 | 2.84 | Dn | nuclear factor I/X (CCAAT-binding transcription factor) |
| TEF | 0.008406 | 0.156835 | 2.84 | Dn | thyrotrophic embryonic factor |
| THNSL2 | 0.001099 | 0.038672 | 2.83 | Dn | threonine synthase-like 2 (S, cerevisiae) |
| FITM2 | 0.001169 | 0.040498 | 2.83 | On | fat storage-inducing transmembrane protein 2 |
| LRRC10 | 0.000583 | 0.023517 | 2.8 | Dn | leucine rich repeat containing 10 |
| GPT | 0.000721 | 0.027858 | 2.79 | Dn | glutamic-pyruvate transaminase (alanine aminotransferase) |
| MGAT5 | 0.002224 | 0.064689 | 2.78 | Dn | mannosyl (alpha-1,6-)-glycoprotein beta-1,6-N-acetyl-glucosaminyltransferase |
| TMEM205 | 0.006892 | 0.13893 | 2.78 | Dn | transmembrane protein 205 |
| NOTCH3 | 0.001918 | 0.05788 | 2.74 | Dn | notch 3 |
| Q5BMM8_CANFA | 0.006717 | 0.135999 | 2.74 | Dn | Uncharacterized protein |
| LIPE | 0.001859 | 0.056696 | 2.74 | Dn | lipase, hormone-sensitive |
| ADCY3 | 0.006958 | 0.139239 | 2.74 | Dn | adenylate cyclase 3 |
| CCND1 | 0.009971 | 0.173147 | 2.73 | Dn | cyclin D1 |
| FBXO40 | 0.004293 | 0.102242 | 2.72 | Dn | F-box protein 40 |
| JPH2 | 0.0017 | 0.053642 | 2.7 | Dn | junctophilin 2 |
| NPR3 | 0.003582 | 0.089802 | 2.7 | Dn | natriuretic peptide receptor C/guanylate cyclase C (atrionatrinretic peptide receptor C) |
| PRICKLE1 | 0.002636 | 0.073118 | 2.7 | Dn | prickle homolog 1 (Drasophila) |
| HMCN1 | 0.001858 | 0.056696 | 2.68 | Dn | hemicentin 1 |
| MLYCD | 0.000804 | 0.030133 | 2.68 | Dn | malonyl-CoA decarboxylase |
| COL14A1 | 0.001219 | 0.041699 | 2.67 | Dn | collagen, type XIV, alpha 1 |
| CD248 | 0.008679 | 0.160448 | 2.67 | Dn | CD248 molecule, endosialin |
| ADCY5 | 0.001641 | 0.052251 | 2.67 | Dn | adenylate cyclase type 5 |
| MAP4K2 | 0.009813 | 0.171266 | 2.66 | Dn | nitrogen-activated protein kinase kinase kinase kinase 2 |
| USP9X | 0.004936 | 0.111565 | 2.65 | Dn | ubiquitin specific peptidase 9, X-linked |
| TAB 1 | 0.005336 | 0.118293 | 2.63 | Dn | TGF-beta activated kinase I/MAP3K7 binding protein 1 |
| GJA3 | 0.00491 | 0.111354 | 2.62 | Dn | gap junction protein, alpha 3, 46kDa |
| TBC1D2B | 0.007717 | 0.149102 | 2.61 | Dn | TBC1 domain family, member 2B |
| SH3BP5 | 0.002422 | 0.068726 | 2.61 | Dn | SH3-domain binding protein 5 (BTK-associated) |
| PLCE1 | 0.004987 | 0.112171 | 2.59 | Dn | 1-phosphatidylinositol-4,5-bisphosphate phosphodiesterase epsilon-1 |
| CLN6 | 0.004186 | 0.101072 | 2.59 | Dn | ceroid-lipofuscinosis neuronal protein 6 homolog |
| RNF128 | 0.007844 | 0.150069 | 2.58 | Dn | ring finger protein 128, E3 ubiquitin protein ligase |
| GM2A | 0.004835 | 0.11061 | 2.57 | Dn | GM2 ganglioside activator |
| LARS2 | 0.00427 | 0.102081 | 2.56 | Dn | leucyl-tRNA synthetase 2, mitochondrial |
| SLC25A34 | 0.004261 | 0.102081 | 2.55 | Dn | solute carrier family 25, member 34 |
| PRR12 | 0.005838 | 0.125586 | 2.55 | Dn | proline rich 12 |
| PRKAR2B | 0.008163 | 0.153972 | 2.55 | Dn | protein kinase, cAMP-dependent, regulatory, type II, beta |
| CASZ1 | 0.008859 | 0.162645 | 2.55 | Dn | castor zinc finger 1 |
| MAPK12 | 0.007291 | 0.142796 | 2.54 | Dn | mitogen-activated protein kinase 12 |
| NRP1 | 0.001863 | 0.056696 | 2.53 | Dn | neuropilin 1 |
| PTPRE | 0.008796 | 0.162096 | 2.53 | Dn | protein tyrosine phosphatase, receptor type, E |
| NDST1 | 0.009455 | 0.16819 | 2.53 | Dn | N-deacetylase/N-sulfotransferase (heparan glucosaminyl) 1 |
| TGFB3 | 0.006699 | 0.135889 | 2.52 | Dn | Transforming growth factor beta 3 |
| MPI | 0.001 363 | 0.045379 | 2.52 | Dn | mannose phosphate isomerase |
| IDB1 | 0.004732 | 0. 109918 | 2.52 | Dn | isocitrate dehydrogenase 1 (NADP+), soluble |
| PLXND1 | 0.002294 | 0.06645 | 2.5 | Dn | plexin DI |
| ITIH5 | 0.007766 | 0.149341 | 2.5 | Dn | inter-alpha-trypsin inhibitor heavy chain family, member 5 |
| C170rf28 | 0.002556 | 0.071333 | 2.49 | Dn | chromosome 17 open reading frame 28 |
| NR2F6 | 0.009163 | 0.166456 | 2.48 | Dn | nuclear receptor subfamily 2, group F, member 6 |
| PAK6 | 0.009519 | 0.168922 | 2.47 | Dn | p21 protein (Cdc42/Rac)-activated kinase 6 |
| SLC40A1 | 0.02301 | 0.066518 | 2.47 | Dn | solute carrier family 40 (iron-regulated transporter), member 1 |
| KLF11 | 0.002844 | 0.077432 | 2.46 | Dn | Kruppel-like factor 11 |
| UACA_CANFA | 0.004944 | 0.111576 | 2.46 | Dn | Uveal antoantigen with coiled-coil domains and ankyrin repeats |
| DGCR2 | 0.006645 | 0.135537 | 2.44 | Dn | DiGeorge syndrome critical region gene 2 |
| SLX4 | 0.006629 | 0.135418 | 2.44 | Dn | SLX4 structure-specific endonuclease subunit homolog (S. cerevisiae) |
| TMC6 | 0.006571 | 0.134969 | 2.44 | Dn | transmembrane channel-like 6 |
| EHMT2 | 0.005812 | 0.125586 | 2.43 | Dn | euchromatic histone-lysine N-methyltransferase 2 |
| MYH8_CANFA | 0.007341 | 0.143561 | 2.43 | Dn | Myosin-8 |
| KIAA1467 | 0.00231 | 0.066633 | 2.43 | Dn | KIAA1467 |
| GNB3 | 0.002092 | 0.061232 | 2.42 | Dn | guanine nucleotide-binding protein G(I)/G(S)/G(T) subunit beta-3 |
| FAM115A | 0.00325 | 0.083762 | 2.42 | Dn | family with sequence similarity 115, member A |
| MACROD1 | 0.003899 | 0.095662 | 2.42 | Dn | MACRO domain containing 1 |
| BCL7A | 0.008567 | 0.15897 | 2.41 | Dn | B-cell CLL/lymphoma 7A |
| TOM1 | 0.002655 | 0.073352 | 2.41 | Dn | target of mybl (chicken) |
| SPG7 | 0.002348 | 0.067301 | 2.4 | Dn | spastic paraplegia 7 (pure and complicated autosomal recessive) |
| novel gene | 0.003655 | 0.091129 | 2.38 | Dn | Uncharacterized protein |
| NKX2-5 | 0.002877 | 0.0777 | 2.37 | Dn | homeobox protein Nkx-2.5 |
| RASGRP3 | 0.002972 | 0.079258 | 2.37 | Dn | RAS guanyl releasing protein 3 (calcium and DAG-regulated) |
| MEOX2 | 0.005486 | 0.120642 | 2.36 | Dn | mesenchyme homeobox 2 |
| PDZRN3 | 0.009265 | 0.166811 | 2.36 | Dn | PDZ domain containing ring finger 3 |
| EPMP1 | 0.009646 | 0.169604 | 2.36 | Dn | endoplasmic reticulum metallopeptidase |
| PDGFB_CANFA | 0.008436 | 0.15697 | 2.34 | Dn | Platelet-derived growth factor subunit B |
| SREBF2 | 0.005014 | 0.1124 | 2.33 | Dn | sterol regulatory element binding transcription factor 2 |
| PLXNA1 | 0.006141 | 0.130071 | 2.31 | Dn | plexin A |
| TACO1 | 0.009949 | 0.172976 | 2.3 | Dn | translational activator of mitochondrially encoded cytochrome c oxidase 1 |
| C5orf4 | 0.007603 | 0.147649 | 2.3 | Dn | chromosome 5 open reading frame 4 |
| PPPIR3A | 0.009267 | 0.166811 | 2.3 | Dn | protein phosphatase I, regulatory subunit 3A |
| PDIA2 | 0.0046,41 | 0.108264 | 2.3 | Dn | protein disulfide isomerase family A, member 2 |
| NID1 | 0.006488 | 0.134321 | 2.29 | Dn | nidogen 1 |
| ABHD6 | 0.007847 | 0.150069 | 2.29 | Dn | abhydroiase domain containing 6 |
| PLA2G4A | 0.007923 | 0.150883 | 2.29 | Dn | phospholipase A2, group IVA (cytosolic, calcium-dependent) |
| RAPSN | 0.00652 | 0.134511 | 2.24 | Dn | receptor-associated protein of the synapse |
| NRID2 | 0.006371 | 0,132885 | 2.24 | Dn | nuclear receptor subfamily I, group D, member 2 |
| GATA4_CANFA | 0.005836 | 0.125586 | 2.23 | Dn | Transcription factor GATA-4 |
| KIAA 1462 | 0.006433 | 0.133387 | 2.21 | Dn | KIAA1462 |
| SLC41A1 | 0.008103 | 0.153037 | 2.18 | Dn | solute carrier family 41, member 1 |
| ΛPOPL5 | 0.008681 | 0.1 60448 | 2.18 | Dn | apolipoprotein I., 5 |
| SERPINF1 | 0.007365 | 0.143638 | 2.17 | Dn | pigment epithelium-derived factor |
| MCAM | 0.00961 | 0.169441 | 2.16 | Dn | melanoma cell adhesion molecule |
| ZNF532 | 0.008805 | 0.1 62096 | 2.13 | Dn | zinc finger protein 532 |
| UNC45B | 0.008969 | 0.164005 | 2.13 | Dn | unc-45 homolog B (C. elegans) |
| RIOK3 | 0.009466 | 0.16819 | 2.1 | Up | RIO kinase 3 (yeast) |
| novel gene | 0.008964 | 0.164005 | 2.12 | Up | Uncharacterized protein |
| NFIL3 | 0.008346 | 0.156135 | 2.15 | Up | nuclear factor, interleukin 3 regulated |
| ODC1 | 0.008086 | 0.153037 | 2.16 | Up | ornithine decarboxylase 1 |
| SGMS1 | 0.006946 | 0.139239 | 2.17 | Up | sphingomyelin synthase 1 |
| novel gene | 0.009288 | 0.166961 | 2.19 | Up | Uncharacterized protein |
| ARF4 | 0.009573 | 0.169235 | 2.19 | Up | ADP-ribosylation factor 4 |
| FDX1 | 0.006249 | 0.131737 | 2.2 | Up | ferredoxin 1 |
| SLC25A33 | 0.00596 | 0.127204 | 2.21 | Up | solute carrier family 25 (pyrimidine nucleotide carrier), member 33 |
| ADSS | 0.006187 | 0.130833 | 2.22 | Up | adenylosuccinate synthase |
| novel gene | 0.006975 | 0.139381 | 2.22 | Up | Uncharacterized protein |
| Q866G8_CANFA | 0.006953 | 0.139239 | 2.22 | Up | elongation factor 1-alpha 1 |
| RELL1 | 0.00705 | 0.140461 | 2.23 | Up | RELT-like 1 |
| novel gene | 0.007222 | 0.142462 | 2.23 | Up | Uncharacterized protein |
| PLK2 | 0.006519 | 0.134511 | 2.23 | Up | polo-like kinase 2 |
| Q866GS_CANFA | 0.006579 | 0.134969 | 2.23 | Up | elongation factor 1-alpha |
| SDC4 | 0.005458 | 0.120209 | 2.23 | Up | syndecan 4 |
| SAMD8 | 0.005363 | 0.118686 | 2.24 | Up | sterile alpha motif domain containing 8 |
| novel gene | 0.006272 | 0.132022 | 2.24 | Up | Uncharacterized protein |
| novel gene | 0.005445 | 0.120121 | 2.24 | Up | Uncharacterized protein |
| RPS6KA2 | 0.00703 | 0.140277 | 2.26 | Up | ribosomal protein S6 kinase, 90kDa, polypeptide 2 |
| novel gene | 0.00638 | 0.132891 | 2.26 | Up | Uncharacterized protein |
| PFN2 | 0.004461 | 0.105678 | 2.26 | Up | profilin 2 |
| novel gene | 0.006873 | 0.138742 | 2.26 | Up | Uncharacterized protein |
| ABRA | 0.006754 | 0.136552 | 2.27 | Up | actin-binding Rho activating protein |
| CXorf36 | 0.005765 | 0.124788 | 2.28 | Up | chromosome X open reading frame 36 |
| novel gene | 0.007267 | 0.142796 | 2.28 | Up | Uncharacterized protein |
| Q8SPM0_CANFA | 0.004289 | 0.102242 | 2.28 | Up | Hypoxia-inducible factor 1 alpha |
| FRIL_CANFA | 0.006336 | 0.132588 | 2.28 | Up | Ferritin light chain |
| BZWI | 0.004735 | 0.109918 | 2.29 | Up | basic leucine zipper and W2 domains 1 |
| MALL | 0.009187 | 0.166457 | 2.29 | Up | mal, T-cell differentiation protein-like |
| RPL21 | 0.009262 | 0.166811 | 2.29 | Up | 608 ribosomal protein L21 |
| novel gene | 0.003916 | 0.095894 | 2.3 | Up | Uncharacterized protein |
| TMEM2 | 0.004213 | 0.101368 | 2.3 | Up | transmembrane protein 2 |
| novel gene | 0.004118 | 0.099615 | 2.31 | Up | Uncharacterized protein |
| novel gene | 0.004628 | 0.108148 | 2.31 | Up | Uncharacterized protein |
| S100A6 | 0.007103 | 0.140924 | 2.31 | Up | S100 calcium binding protein A6 |
| C1QC | 0.006545 | 0.134689 | 2.31 | Up | complement component 1, q subcomponent, C chain |
| TUBA1C | 0.003636 | 0.090821 | 2.31 | Up | tubulin, alpha 1c |
| novel gene | 0.003855 | 0.09497 | 2.32 | Up | Uncharacterized protein |
| FRIL_CANFA | 0.006927 | 0.139239 | 2.32 | Up | Ferritin light chain |
| FSTL3 | 0.004846 | 0.110631 | 2.32 | Up | follistatin-like 3 (secreted glycoprotein) |
| novel gene | 0.004118 | 0.099615 | 2.32 | Up | Uncharacterized protein |
| novel gene | 0.006005 | 0.127722 | 2.33 | Up | Uncharacterized protein |
| CLK 1 | 0.004745 | 0.109956 | 2.34 | Up | CDC-like kinase 1 |
| ELL | 0.00706 | 0.140461 | 2.34 | Up | elongation factor RNA polymerase II |
| novel gene | 0.007282 | 0.142796 | 2.35 | Up | Uncharacterized protein |
| RHOB | 0.003513 | 0.088723 | 2.36 | Up | ras homolog family member B |
| GRAMD3 | 0.00315 | 0.082416 | 2.36 | Up | GRAM domain containing 3 |
| novel gene | 0.006687 | 0.135889 | 2.36 | Up | Uncharacterized protein |
| ALAS1 | 0.005864 | 0.125918 | 2.38 | Up | aminolevulinate, delta-, synthase 1 |
| AQPI_CANFA | 0.007707 | 0.149102 | 2.38 | Up | Aquaporin-1 |
| novel gene | 0.007356 | 0.143638 | 2.38 | Up | Uncharacterized protein |
| ART3 | 0.009168 | 0.166456 | 2.38 | Up | ADP-ribosyltransferase 3 |
| KLHL2 | 0.002469 | 0.069768 | 2.39 | Up | kelch-like 2, Mayven (Drosophila) |
| CCNL1 | 0.002901 | 0.077982 | 2.39 | Up | cyclin L1 |
| UAP1 | 0.002654 | 0.073352 | 2.4 | U p | UDP-N-acteylglucosamine pyrophosphorylase 1 |
| SPSB1 | 0.007166 | 0.141548 | 2.41 | Up | splA/ryanodine receptor domain and SOCS box containing 1 |
| novel gene | 0.004532 | 0.106628 | 2.41 | Up | Uncharacterized protein |
| novel gene | 0.002348 | 0.067301 | 2.41 | Up | Uncharacterized protein |
| TAGLN | 0.004097 | 0.099448 | 2.42 | Up | transgelin |
| novel gene | 0.003735 | 0.092452 | 2.42 | Up | Uncharacterized protein |
| novel gene | 0.006337 | 0.132588 | 2.42 | Up | Uncharacterized protein |
| TMM47_CANFA | 0.002529 | 0.070865 | 2.43 | Up | Transmembrane protein 47 |
| novel gene | 0.004796 | 0.110415 | 2.43 | Up | Uncharacterized protein |
| novel gene | 0.00715 | 0.141548 | 2.43 | Up | Uncharacterized protein |
| ACTB_CANFA | 0.006598 | 0.134969 | 2.43 | Up | Actin, cytoplasmic 1 Actin, cytoplasmic 1, N-terminally processed |
| Q6SLL2_CANFA | 0.00316 | 0.082508 | 2.43 | Up | urokinase-type plasminogen activator precursor |
| NOV | 0.006199 | 0.130884 | 2.44 | Up | nephroblastoma overxpressed |
| PDE4B | 0.005512 | 0.121011 | 2.44 | Up | phosphodiesterase 4B, cAMP-specific |
| OR51 E2 | 0.00301 | 0.079821 | 2.45 | Up | olfactory receptor, family 51, subfamily E, member 2 |
| MCL1 | 0.007079 | 0.140635 | 2.45 | Up | induced myeloid leukemia cell differentiation protein Mcl-1 homolog |
| CLIC1 | 0.004819 | 0.110563 | 2.45 | Up | chloride intracellular channel protein 1 |
| EPHA2 | 0.005742 | 0.124686 | 2.46 | Up | EPH receptor A2 |
| CD22 | 0.006702 | 0.135889 | 2.46 | Up | CD22 molecule |
| SRSF3 | 0.001746 | 0.054143 | 2.47 | Up | serine/arginine-rich splicing factor 3 |
| CNN2 | 0.003006 | 0.079821 | 2.48 | Up | calponin 2 |
| RPF2 | 0.003071 | 0.080969 | 2.48 | Up | ribosome production factor 2 homolog (S. cerevisiae) |
| novel gene | 0.001715 | 0.053747 | 2.5 | Up | Uncharacterized protein |
| O97530_CANFA | 0.003241 | 0.083712 | 2.51 | Up | Tumor necrosis factor receptor p60 |
| novel gene | 0.008101 | 0.153037 | 2.51 | Up | Uncharaeterized protein |
| EMCN | 0.002821 | 0.077093 | 2.51 | Up | endomucin |
| ATP1B3 | 0.003471 | 0.08797 | 2.52 | Up | ATPase, Na+/K+ transporting, beta 3 polypeptide |
| FAM43A | 0.005712 | 0.124232 | 2.53 | Up | family with sequence similarly 43, member A |
| novel gene | 0.00306 | 0.080822 | 2.54 | Up | Uncharacterized protein |
| PDXK | 0.001098 | 0.038672 | 2.54 | Up | pyridoxal (pyridoxine, vitamin B6) kinase |
| novel gene | 0.006357 | 0.13281 | 2.55 | Up | Uncharacterized protein |
| PNPLA8 | 0.001911 | 0.05778 | 2.55 | Up | patatin-like phospholipase containing 8 |
| SH2D3C | 0.00727 | 0.142796 | 2.56 | Up | 5H2 domain containing 3C |
| GSTM3 | 0.006661 | 0.135658 | 2.56 | Up | glutathione S-transferase mu 3 (brain) |
| MMD | 0.001926 | 0.057986 | 2.56 | Up | monocyte to macrophage differentiation-associated |
| CIQA | 0.002735 | 0.075256 | 2.57 | Up | complement component l, q subcomponent, A chain |
| TXN | 0.001869 | 0.056755 | 2.57 | Up | thioredoxin |
| S100A11 | 0.002799 | 0.076736 | 2.57 | Up | S100 calcium binding protein A11 |
| DEGS1 | 0.001073 | 0.038044 | 2.57 | Up | delta(4)-desaturase, sphingolipid 1 |
| TGFBR2 | 0.001158 | 0.040232 | 2.58 | Up | transforming growth factor, beta receptor 11 (70/80kDa) |
| BTG2 | 0.003234 | 0.083712 | 2.59 | Up | BTG family, member 2 |
| RAP1B | 0.001109 | 0.038903 | 2.6 | Up | RAP1B, member of RAS oncogene family |
| novel gene | 0.003568 | 0.089767 | 2.6 | Up | Uncharacterized protein |
| novel gene | 0004194 | 0.101085 | 2.6 | Up | Uncharacterized protein |
| CTTNBP2NL | 0.003821 | 0.09441 1 | 2.61 | Up | CTTNBP2 N-teminal like |
| novel gene | 0.001849 | 0.056696 | 2.61 | Up | Uncharacterized protein |
| IFRD1 | 0.000924 | 0.033794 | 2.63 | Up | interferon-related developmental regulator 1 |
| novel gene | 0.001252 | 0.04245 | 2.64 | Up | Uncharacterized protein |
| RASA2 | 0.009809 | 0.171266 | 2.64 | Up | RAS p21 protein activator 2 |
| SKIL | 0.000927 | 0.033811 | 2.65 | Up | SKI-like oncogene |
| SWAP70 | 0.001146 | 0.039917 | 2.65 | Up | SWAP switching B-cell complex 70kDa subunit |
| TMEM181 | 0.001372 | 0.04557 | 2.65 | Up | transmembrane protein 181 |
| novel gene | 0.008519 | 0.158307 | 2.66 | Up | Uncharacterized protein |
| ILAR | 0.001539 | 0.049943 | 2.66 | Up | interleukin 4 receptor |
| novel gene | 0.000815 | 0.030458 | 2.66 | Up | Uncharacterized protein |
| TLR4 | 0.001656 | 0.052587 | 2.68 | Up | toll-like receptor 4 precursor |
| TUBA1B | 0.00237 | 0.067668 | 2.68 | Up | tubulin, alpha 1b |
| FLNB | 0.000872 | 0.032163 | 2.69 | Up | filamin B, beta |
| COL16A1 | 0.007968 | 0.151537 | 2.7 | Up | collagen, type XVI, alpha 1 |
| NPNT | 0.002061 | 0.061044 | 2.7 | Up | nephronectin |
| AOFA_CANFA | 0.000712 | 0.027567 | 2.71 | Up | Amine oxidase A |
| SLC35D1 | 0.000682 | 0.026865 | 2.71 | Up | solute carrier family 35 (UDP-glucuronic acid/UDP-N-acetylgalactosamine dual transporter), member D1 |
| CORO1A | 0.009834 | 0.171409 | 2.72 | Up | coronin, actin binding protein, 1A |
| CD163 | 0.005115 | 0.114484 | 2.72 | Up | scavenger receptor cysteine-rich type 1 protein M130 precursor |
| ATP 13A3 | 0.001362 | 0.045379 | 2.73 | Up | ATPase type 13A3 |
| RBM3 | 0.001567 | 0.050702 | 2.74 | Up | RNA binding motif (RNP1, RRM) protein 3 |
| THBD | 0.001452 | 0.047682 | 2.75 | Up | thrombomodulin precursor |
| SLC25A25 | 0.000501 | 0.02082 | 2.76 | Up | solute carrier family 25 (mitochondrial carrier; phosphate carrier), member 25 |
| FHL1 | 0.002068 | 0.061044 | 2.76 | Up | four and a half LIM domains 1 |
| novel gene | 0.002356 | 0.067401 | 2.77 | Up | Uncharacterized protein |
| SORL1 | 0.004502 | 0.106281 | 2.78 | Up | sortilin-relaled receptor, L(DLR class) A repeats containing |
| AMIGO2 | 0.00934 | 0.167087 | 2.79 | Up | adhesion molecule with Ig-like domain 2 |
| novel gene | 0.000568 | 0.023099 | 2.8 | Up | Uncharacterized protein |
| ARPC1B | 0.000467 | 0.019592 | 2.8 | Up | actin related protein 2/3 complex, subunit 1B, 41kDa |
| RFX2 | 0.004546 | 0.106779 | 2.81 | Up | regulatory factor X, 2 (influences HLA class II expression) |
| COTL1 | 0.003087 | 0.08122 | 2.83 | Up | coactosin-like 1 (Dictyostelium) |
| SLC3A1 | 0004513 | 0.106365 | 2.83 | Up | neutral and basic amino acid transport protein rBAT |
| PLAGL1 | 0.004601 | 0.107703 | 2.84 | Up | pleiomorphic adenoma gene-like 1 |
| FAM188A | 0.004021 | 0.097943 | 2.85 | Up | family with sequence similarity 188, member A |
| FRMD8 | 0.000481 | 0.020056 | 2.85 | Up | FERM domain containing 8 |
| novel gene | 0.009562 | 0.169235 | 2.85 | Up | Uncharacterized protein |
| TAGLN2 | 0.000311 | 0.014471 | 2.85 | Up | transgelin 2 |
| RHOU | 0.000354 | 0.016031 | 2.85 | Up | ras homolog family member U |
| C9orf153 | 0.006941 | 0.139239 | 2.86 | Up | chromosome 9 open reading frame 153 |
| IL33 | 0.003382 | 0.086517 | 2.87 | Up | interleukin-33 precursor |
| MSX1 | 0.0031 84 | 0.082815 | 2.87 | Up | msh homeobox 1 |
| PM20D2 | 0.00026 | 0.012607 | 2.88 | Up | peptidase M20 domain containing |
| ESM1 | 0.008207 | 0.154596 | 2.89 | Up | endothelial cell-specific molecule 1 |
| DUSP6 | 0.000421 | 0.017961 | 2.9 | Up | dual specificity phosphatase 6 |
| NDRG1 | 0.00024 | 0.011994 | 2.91 | Up | N-myc downstream regulated 1 |
| GNE | 0.001639 | 0.052251 | 2.91 | Up | glucosamine (UDP-N-acetyl)-2-epimerase/N-acetylmannosamine kinase |
| UGDH | 0.00032 | 0.014787 | 2.92 | Up | UDP-glucose 6-dehydrogenase |
| novel gene | 0.002181 | 0.063583 | 2.93 | Up | Uncharacterized protein |
| CRISPLD2 | 0.000242 | 0.012053 | 2.93 | Up | cysteine-rich secretory protein LCCL domain containing 2 |
| GPR4 | 0.009311 | 0.167087 | 2.93 | Up | G protein-coupled receptor 4 |
| SLC1A5 | 0.004583 | 0.107465 | 2.94 | Up | solute carrier family 1 (neutral amino acid transporter), member 5 |
| SMAD6 | 0.001305 | 0.043867 | 2.94 | Up | SMAD family member 6 |
| SOAT1 | 0.008319 | 0.156048 | 2.94 | Up | sterol O-acyltransferase 1 |
| GLIPR2 | 0.000455 | 0.019132 | 2.94 | Up | GLI pathogenesis-related 2 |
| ARPC3 | 0.008043 | 0.152544 | 2.94 | Up | |
| novel gene | 0.004803 | 0.110415 | 2.95 | Up | Uncharacterized protein |
| RASSF5 | 0.00926 | 0.166811 | 2.97 | Up | Ras association (RalGDS/AF-6) domain family member 5 |
| C1QB | 0.000474 | 0.019824 | 2.97 | Up | complement component 1, q subcomponent, B chain |
| ASB9 | 0.003684 | 0.091689 | 2.98 | Up | ankyrin repeat and SOCS box containing 9 |
| TRMT11 | 0.001636 | 0.052251 | 2.98 | Up | tRNA methyltransferase 1 I homolog (S. cerevisiae) |
| RND3 | 0.000144 | 0.007925 | 2.99 | Up | Rho family GTPase 3 |
| PLP2 | 0.000358 | 0.016122 | 2.99 | Up | proteolipid protein 2 |
| ABCC4 | 0.004761 | 0.110149 | 3 | Up | multidrug resistance-associated protein 4 |
| SOX9 | 0.001641 | 0.052251 | 3.02 | Up | transcription factor SOX-9 |
| NR4A1_ CANFA | 0.001308 | 0.043867 | 3.04 | Up | Nuclear receptor subfamily 4 group A member 1 |
| AP3S1 | 0.00594 | 0.126972 | 3.07 | Up | adaptor-related protein complex 3, sigma 1 subunit |
| SIPR1 | 0.000208 | 0.01078 | 3.07 | Up | sphingosine-1-phosphate receptor 1 |
| novel gene | 0.008781 | 0.162077 | 3.08 | Up | Uncharacterized protein |
| NPPB | 0,009693 | 0.170043 | 3.08 | Up | natrinretic peptide B |
| ACTG1 | 0,000355 | 0.016031 | 3.09 | Up | Uncharaeterized protein |
| novel gene | 0000166 | 0.009004 | 3.1 | Up | Uncharacterized protein |
| SDF2L1 | 0001528 | 0.049701 | 3.11 | Up | stromal cell-derived factor 2-like 1 |
| DOK2 | 0.009009 | 0.164522 | 3.13 | Up | docking protein 2, 56kDa |
| IER3 | 0.000387 | 0.016769 | 3.14 | Up | immediate early response 3 |
| EMR1 | 0.001663 | 0.052587 | 3.16 | Up | EGF-like module-containing mucin-like hormone receptor-like 1 precursor |
| IRS2 | 0.0001 | 0.006055 | 3.17 | Up | insulin receptor substrate 2 |
| GEM | 0.005651 | 0.123285 | 3.17 | Up | GTP binding protein overexpressed in skeletal muscle |
| TAF7L | 0.004972 | 0.112006 | 3.17 | Up | TAF7-like RNA polymerase II, TATA box binding protein (TBP)-associated factor, 50kDa |
| TMEMI76A | 0.000107 | 0.0063 | 3.18 | Up | transmembrane protein 176A |
| ANKRD37 | 0.002012 | 0.060059 | 3.18 | Up | ankyrin repeat domain 37 |
| novel gene | 0.007165 | 0,141548 | 3.19 | Up | Uncharactenzed protein |
| CYR61 | 0.000214 | 0.010988 | 3.19 | Up | eysteitie-rich, angiogenic inducer, 61 |
| FAM110D | 0.001705 | 0.053666 | 3.19 | Uρ | family with sequence similarity 110, member D |
| QTUD1 | 0.000271 | 0.012961 | 3.2 | Up | OTU domain containing 1 |
| ROBO4 | 9.18E-05 | 0.005649 | 3.2 | Up | roundabout, axon guidance receptor, homolog 4 (Drosophila) |
| BHLHE40 | 7.14E-05 | 0.00459 | 3.2 | Up | basic helix-loop-helix family, member e40 |
| RALB | 6.7IE-05 | 0.004384 | 3.22 | Up | v-ral simian leukemia viral oncogene homolog B (ras related; GTP binding protein) |
| KDM6B | 0,000373 | 0.016316 | 3.22 | Up | lysine (K)-specific demethylase 6B |
| PLK3 | 0.005395 | 0.1192 | 3.26 | Up | polo-like kinase 3 |
| ROR 1 | 0.009656 | 0. 169604 | 3.26 | U p | receptor tyrosine kinase-like orphan receptor 1 |
| SLCO1C1 | 0.002381 | 0.067825 | 3.26 | U p | solute carrier organic anion transporter family, member 1C1 |
| PIK3R5 | 0,005882 | 0.126031 | 3.27 | Up | phosphoinosiride-3-kinase, regulatory subunit 5 |
| TRIB1 | 0.000248 | 0.012204 | 3.28 | Up | tribbies homolog 1(Drosophila) |
| ADML _ CANFA | 7.31E-05 | 0.004647 | 3.29 | Up | ADM Adrenomedullin Proadrenorrsedullin N-2O terminal peptide |
| novel gene | 0.000118 | 0.006735 | 3.31 | Up | Uncharacterized protein |
| LRRC8C | 7.73E-05 | 0004843 | 3.32 | Up | leucine rich repeat containing 8 family, member C |
| CXCL16 | 0.009651 | 0.169604 | 3.33 | Up | chemokine (C.X.C motif) ligand 16 |
| novel gene | 6.74E-05 | 0.004384 | 3.36 | Up | Uncharacterized protein |
| PXDC 1 | 0.001253 | 0.04245 | 3.39 | Up | PX domain containing 1 |
| AP1S3 | 3.44E-05 | 0.003621 | 3.4 | Up | adaptor-related protein complex 1, sigma 3 subunit |
| KLF4 | 0.001615 | 0.051927 | 3.4 | Up | Kruppel-like factor 4 (gut) |
| TSPAN19 | 0.007897 | 0.150804 | 3,41 | Up | tetraspanin 19 |
| RASSF1 | 0.000425 | 0.018084 | 3.44 | Up | Ras association (Ral3DS/AF-6) domain family member 1 |
| TYROBP | 0.002824 | 0.077093 | 3.44 | Up | TYRO protein tyrosine kinase-binding protein precursor |
| DEF6 | 0.000649 | 0.025843 | 3.44 | Up | differentially expressed in FDCP 6 homolog, (mouse) |
| STAT3 | 4.72E-05 | 0.003281 | 3.45 | Up | signal transducer and activator of transcription 3 (acute-phase response factor) |
| NABP1 | 0.009343 | 0.167087 | 3.45 | Up | nucleic acid binding protein 1 |
| VWF | 0.000261 | 0.012607 | 3.45 | Up | von Willebrand factor precursor |
| TNFAIP3 | 0.001662 | 0.052587 | 3.45 | Up | minor necrosis factor, alpha-induced protein 3 |
| ERO1L | 4.88E-05 | 0.003331 | 3.48 | Up | ERO1-like (S. cerevisiae) |
| CYP1B1 | 0.000333 | 0.015323 | 3.5 | Up | cyrochrome P450 1B1 |
| RHOJ | 0.000114 | 0.006565 | 3.5 | Up | ras homolog family member J |
| C10orf10 | 0.002992 | 0.079651 | 3.53 | Up | chromosome 10 open reading frame 10 |
| CPS1 | 0.003276 | 0.084262 | 3.54 | Up | carbamoyl-phosphate synthase 1, mitochondrial |
| KLF5 | 0.00036 | 0.016122 | 3.54 | Up | Kruppel-like factor 5 (intestinal) |
| ELOVL7 | 0.000118 | 0.006733 | 3.55 | Up | ELOVL fatty acid elongase 7 |
| ARID5A | 1.77E-05 | 0,00143 | 3.55 | Up | AT rich interactive domain 5A (MRFI-like) |
| novel gene | 2.74E-05 | 0.002079 | 3.58 | Up | Uncharacterized protein |
| ALAS2 | 0.000201 | 0.010497 | 3.59 | Up | aminolevulinate, delta-, synthase 2 |
| ETS2 | 1.15E-05 | 0.000976 | 3.59 | Up | v-ets erythroblastosis virus E26 oncogene homolog 2 (avian) |
| novel gene | 1.39E-05 | 0.001154 | 3.61 | Up | Uncharacterized protein |
| KLHL29 | 0.006292 | 0.132067 | 3.61 | Up | kelch-like 29 (Drosophila) |
| novel gene | 0.000605 | 0.024193 | 3.61 | Up | Uncharacterized protein |
| RAH4 | 0.002868 | 0.0777 | 3.62 | Up | retinoic acid induced 14 |
| novel gene | 0.005228 | 0.116634 | 3.63 | Up | Uncharacterized protein |
| ABRACL | 0.000169 | 0.009124 | 3.64 | Up | ABRA C-terminal like |
| EDNRB | 8,66E-06 | 0.000796 | 3.66 | Up | endothelin B receptor precursor |
| ARNTL | 0.001214 | 0.041655 | 3.68 | Up | aryl hydrocarbon receptor nuclear translocator-like |
| TMEMI82 | 4.73E.05 | 0.003281 | 3.69 Up | | transmembrane protein 182 |
| ACTN1 | 0.000249 | 0.012234 | 3.71 | U p | actinin, alpha 1 |
| MTHFD2 | 0.004931 | 0.111565 | 3.72 | Up | methylenetetrahydrofolate dehydrogenase (NADP+ dependent) 2. methenyltetrahydrofolate cyclohydrolase |
| EKBP5 | 2.06E-05 | 0.001625 | 3.76 | Up | FK506 binding protein 5 |
| CTHRC1 | 0.004805 | 0.110415 | 3.77 | Up | collagen triple helix repeat containing 1 |
| DLL1 | 0.000192 | 0.010166 | 3.77 | Up | delta-like 1 (Drosophila) |
| LY9 | 0.007838 | 0.150069 | 3.8 | Up | lymphocyte antigen 9 |
| ERRFSII | 0.00052 | 0.021393 | 3,81 | Up | ERRB receptor feedback inhibitor 1 |
| PTGI R | 0.000967 | 0.034794 | 3.81 | Up | prostaglandin. 12 (prostacyclin) receptor (IP) |
| TEAD4 | 0.000283 | 0.01338 | 3.81 | Up | TEA domain family member 4 |
| HAPLN3 | 0.001071 | 0.038044 | 3.82 | Up | hyaluronan and proteoglycan link protein 3 |
| NPPA | 0.000256 | 0.012473 | 3.87 | Up | Natriuretic peptides A Atrial natriuretic factor |
| novel gene | 0.000701 | 0.027381 | 3.91 | Up | Uncharacterized protein |
| C14orf37 | 0.005297 | 0.117807 | 3.93 | Up | chromosome 14 open reading frame 37 |
| ST6GALNAC3 | 0.00024 | 0.01 1994 | 3.94 | Up | ST6 (alpha-N-acetyl-neuraminyl-2,3-beta-galactosyl-1,3)-N-acetylgalactosaminide alpha-2,6-sialyltransferase 3 |
| CSF1 | 0.00021 | 0.010835 | 3.95 | Up | colony stimulating factor 1 (macrophage) |
| KIAA0556 | 0.002606 | 0.072445 | 3.96 | Up | KIAA0556 |
| SEMA7A | 9.04E-06 | 0.000911 | 3.97 | Up | semaphorin 7A, GP1 membrane anchor (John Milton Hagen blood group) |
| novel gene | 0.000756 | 0.028806 | 4 | Up | Uttcharacterized protein |
| FGR | 0.000107 | 0.0063 | 4.05 | Up | Gardner-Rasheed feline sarcoma viral (v-fgr) oncogene homolog |
| KRT80 | 0.009586 | 0.169239 | 4.05 | Up | keratin 80 |
| LRRC32 | 0.000791 | 0.029868 | 4.09 | Up | leucine rich repeat containing 32 |
| HK3 | 0.009372 | 0.167 166 | 4.09 | Up | hexokinase 3 (white cell) |
| TPM4 | 1.56E-06 | 0.000193 | 4.15 | Up | tropomyosin 4 |
| UGCG | 9.08E-07 | 0.000128 | 4.15 | Up | ceramide glucosyltransferase |
| ENTPD3 | 0.000947 | 0.034286 | 4.16 | Up | ectonucleoside triphosphate diphosphohydrolase 3 |
| SLC20A1 | 1.29E-06 | 0.000163 | 4.18 | Up | solute carrier family 20 (phosphate transporter), member 1 |
| SIG1RR | 0.003525 | 0.088848 | 4.2 | Up | single immunoglobulin and toll-interieukin 1 receptor (TIR) domain |
| ADAMTS1 | 2.07E-06 | 0.000245 | 4.23 | Up | ADAM metallopeptidase with thrombospondin type 1 motif, 1 |
| LYVE1 | 9.24E-06 | 0.000811 | 4.23 | Up | lymphatic vessel endothelial hyaluronan receptor 1 |
| NFKBIA | 2.95E-06 | 0.000328 | 4.25 | Up | nuclear factor of kappa light polypeptide gene enhancer in B-cells inhibitor, alpha |
| novel gene | 0.00071 | 0.027567 | 4.26 | Up | Uncharacterized protein |
| IL1R2 | 0.00035 | 0.01596 | 4.28 | Up | interleukin 1 receptor, type II |
| SPINK4 | 0.001573 | 0.050785 | 4.3 | Up | serine peptidase inhibitor, Kazal type 4 |
| CLlC2 | 4.43E-06 | 0.000463 | 4.35 | Up | chloride intracellular channel protein 2 |
| PRSS23 | 6,51E-06 | 0.000634 | 4.39 | Up | protease, serine, 23 |
| O97702 CANFA | 4.38E-05 | 0.003081 | 4.41 | Up | integrin beta-3 precursor |
| ARL4A | 1.07E-05 | 0.000919 | 4.41 | Up | ADP-ribosylation factor-like 4A |
| ASNS | 0.000225 | 0.011423 | 4.45 | Up | asparagine synthetase (glutamine-hydrolyzing) |
| PMEPA1 | 4.29E-06 | 0.000452 | 4.52 | Up | prostate transmembrane protein, androgen induced 1 |
| IER5L | 6.07E-05 | 0.004003 | 4.57 | Up | immediate early response 5-like |
| E5D812_CANFA | 0.00059 | 0.023702 | 4.62 | Up | Uncharacterized protein |
| SGK1 | 0.000197 | 0.010326 | 4.68 | Up | serum/glucocorticoid regulated kinase 1 |
| novel gene | 0.005164 | 0.115391 | 4.72 | Up | Uncharacterized protein |
| novel gene | 0.000517 | 0.021373 | 4.74 | Up | Uncharacterized protein |
| CD300C | 0.000189 | 0.010002 | 4.77 | Up | CD300c molecule |
| PLEK CANFA | 0.000525 | 0.021472 | 4.77 | Up | Pleckstrin |
| EMB | 0.006527 | 0.134511 | 4.78 | Up | embigin |
| MTHFD1L | 1.95E-05 | 0.001553 | 4.79 | Up | methylenetetrahydrofolate dehydrogenase (NADP+ dependent) 1-like |
| SLC16A3 | 0.000761 | 0.028903 | 4.84 | Up | solute carrier family 16, member 3 (monocarboxylic acid transporter 4) |
| SLCO6A1 | 0.005335 | 1 | 4.85 | Up | solute carrier anion transporter family, member 6A1 |
| PG F. | 2.20E-05 | 0.001713 | 4.86 | Up | placental growth factor |
| SOX 17 | 1.28E-06 | 0.000163 | 4 86 | Up | SRY (sex determining region Y)-box 17 |
| CCDC172 | 0.001092 | 0.038598 | 4.89 | Up | coiled-coil domain containing 172 |
| VCAN | 3.47E-07 | 5.77E-05 | 4.9 | Up | versican |
| NFKBIZ | 7.63E-06 | 0.000721 | 4.98 | Up | nuclear factor of kappa light polypeptide gene enhancer in B-cells inhibitor, zeta |
| SNAI1 | 9.96E-05 | 0.006052 | 4.99 | Up | snail homolog 1 (Drosophila) |
| VIPR2 | 0.000952 | 0.034378 | 4.99 | Up | vasoactive intestinal peptide receptor 2 |
| C13orf33 | 7.49E-08 | 1.56E-05 | 5 | Up | chroroosome 13 open reading frame 33 |
| MUC20 | 0.007959 | 1 | 5.02 | Up | mucin 20, cell surface associated |
| IL15 | 6.58E-06 | 0.000634 | 5.04 | Up | interleukin-15 |
| SPP1 | 0.000179 | 0.009552 | 5.04 | Up | secreted phosphoprotein 1 |
| novel gene | 2.14E-05 | 0.001673 | 5.06 | Up | Uncharacserized protein |
| GFPT2 | 4.14E-07 | 6.72E-05 | 5.08 | Up | glutamine-fructose-6-phosphate transaminase 2 |
| novel gene | 2.28E-05 | 0.001754 | 5.1 | Up | Uncharacterized protein |
| NFE2 | 0.006857 | 1 | 5.19 | Up | nuclear factor (erythroid-derived 2), 45kDa |
| | | | | | |
| FGF7_CANFA | 8.56E-07 | 0.000122 | 5.21 | Up | Fibroblast growth factor 7 |
| ICAM1 | 4.23E-07 | 6.79E-05 | 5.22 | Up | intercellular adhesion molecule I precursor |
| MYC | 2.09E-07 | 3.75E-05 | 5.24 | Up | myc proto-onogene protein |
| EGR1 | 9.79E-07 | 0.000136 | 5.27 | Up | early growth response 1 |
| PVR | 6.30E-06 | 0.000629 | 5.29 | Up | poliovirus receptor |
| ICOSLG | 8.48E-06 | 0.000785 | 5.3 | Up | inducible T-cell co-stimulator ligand |
| HGF_CANFA | 3.88E-05 | 0.002797 | 5.31 | Up | Hepatocyte growth factor Hepatocyte growth factor alpha chain Hepatocyte growth factor beta chain |
| G0ZS87_CANFA | 0.009138 | 0.166456 | 5.39 | Up | Triggering receptor expressed on myeloid cells 1 |
| OSMR | 1.46E-08 | 3.94E-06 | 5.4 | Up | oncostatin M receptor |
| AKAP12 | 1.62E-08 | 4.20E-06 | 5.42 | Up | A kinase (PRKA) anchor protein 12 |
| E7ECW0_ CANFA | 0.000236 | 0.011867 | 5.43 | Up | arachidonate 5-lipoxygenase |
| THBS4 | 1.91E-06 | 0.000219 | 5.45 | Up | thrombospondin 4 |
| novel gene | 0.003896 | 0.095662 | 5.48 | Up | Uncharacterized protein |
| CA4 | 1.06E-08 | 2.93E-06 | 5,5 | Up | carbonic anhydrase IV |
| MYOC_CANFA | 5.55E-06 | 0.000567 | 5.55 | Up | Myocilin |
| BCL3 | 1.93E-05 | 0.001547 | 5.59 | Up | B-cell CLL/lymphoma 3 |
| LOX | 2.44E-07 | 4.32E-05 | 5.61 | Up | lysyl oxidase |
| Q683K8_CANFA | 2.35E-08 | 5.59E-06 | 5.66 | Up | Puiative cyclin-dependent kinase inhibitor 1A. (P21/WAF1/CIP1) |
| FGL2 | 2.88E-07 | 5.03E-05 | 5.68 | Up | fibrinogen-like 2 |
| novel gene | 0.001066 | 0.037986 | 5.7 | Up | Uncharacterized protein |
| NOS3 | 4.03E-09 | 1.28E-06 | 5.72 | Up | nitric oxide synthase, endothelial |
| HK2 | 6.93E-06 | 0.00066 | 5.76 | Up | hexokinase 2 |
| LY86 | 0.008665 | 0.160448 | 5.85 | Up | lymphocyte antigen 86 |
| MYOF | 3.02E-07 | S.21E-05 | 5.85 | Up | myoferlin |
| SEMA3F | 1.84R-06 | 0.000223 | 5.85 | Up | sema domain, immunoglobulin domain (Ig), short basic domain, secreted, (semaphorin) 3F |
| RETN | 0.000591 | 0.023702 | 5.9 | Up | resistin |
| ACER2 | 4.09E-09 | 1.28E-06 | 5.91 | Up | alkaline ceramidase 2 |
| DRAM1 | 0.000748 | 0.028562 | 5.95 | Up | DNA-damage regulated autophagy modulator 1 |
| SRGN | 1.03E-06 | 0.000139 | 5.99 | Up | serglycin |
| SERPINE2 | 2.63E-08 | 6.14E-06 | 6.02 | Up | serpin peptidase inhibitor, clade E (nexin, plasminogen activator inhibitor type 1), member 2 |
| PLED1 | 2.25E-05 | 0.001747 | 6.02 | Up | phospholipase B doamin containing 1 |
| JUNB | 8.84E-09 | 2.49E-06 | 6.07 | Up | jun B proto-oncogene |
| TREM2 | 0.004876 | 0.11079 | 6.15 | Up | triggering receptor expressed on myeloid cells 2 |
| WDR89 | 1.52E-05 | 0.001245 | 6.17 | Up | WD repeat domain 89 |
| SNORA25 | 0.004784 | 0.110415 | 6.25 | Up | Small nucleolar RNA SNORA25 |
| novel gene | 1.26E-07 | 2.38E-05 | 6.25 | Up | Uncharacterized protein |
| IRF4 | 0.000401 | 0.01735 | 6.26 | Up | interferon regulatory factor 4 |
| IGFBP2 | 0.000107 | 0.0063 | 6.28 | Up | insulin-like growth factor binding protein 2, 36kDa |
| BSPRY | 0.000574 | 0.023262 | 6.33 | Up | B-box and SPRY domain containing |
| GADD45B | 4.85E-09 | 1.45E-06 | 6.4 | Up | growth arrest and DNA-damage-inducible, beta |
| DKK2 | 8,82E-10 | 3.20E-07 | 6.4 | Up | dickkopf 2 homolog (Xenopus laevis) |
| HIVEP3 | 0.002707 | 1 | 6.43 | Up | human immunodeficiency virus, type 1 enhancer binding protein 3 |
| novel gene | 9.79E-06 | 0.000854 | 6.45 | Up | Uncharacterized protein |
| PLAC8 | 0.000679 | 0.026836 | 6.45 | Up | placenta-specific 8 |
| TKTL1 | 5.63E-06 | 0.000571 | 6.51 | Up | transketolase-like 1 |
| C17orf64 | 0.001711 | 0.053733 | 6.54 | Up | chromosome 17 open reading frame 64 |
| LRRC25 | 0.003395 | 0.086696 | 6.55 | Up | leucine rich repeat containing 25 |
| PAD14 | 0.000804 | 0.030133 | 6.71 | Up | peptidyl arginine deiminase, type IV |
| MAPK13 | 7.81E-06 | 0.000732 | 6.71 | Up | mitogen-activated protein kinase 13 |
| novel gene | 6.56E-06 | 0.000634 | 6.76 | Up | Uncharacterized protein |
| SERPINA3 | 0.000863 | 0.032003 | 6.78 | Up | serpin peptidase inhibitor, clade A (alpha-1 antiproteinase, antitrypsin), member 3 |
| CTGF | 8.51E-10 | 3.17E-07 | 6.85 | Up | connective tissue growth factor |
| C5orf62 | 0.000244 | 0.012067 | 6.86 | Up | chromosome 5 open reading frame 62 |
| novel gene | 0.001034 | 0.036932 | 6.94 | Up | Uncharacterized protein |
| CSRNP1 | 6.97E-08 | 1.48E-05 | 7.11 | Up | cysteine-serine-rich nuclear protein 1 |
| FCGRIA | 2.36E-06 | 0.000274 | 7.13 | Up | high affinity immunoglobulin gamma Fc receptor I precursor |
| SLC26A7 | 9.72E-05 | 0.005933 | 7.16 | Up | solute carrier family 26, member 7 |
| PRPH | 2.48E-06 | 0.000183 | 7.17 | Up | peripherin |
| SIK1 | 1.31E-09 | 4.53E-07 | 7.17 | Up | salt-inducible kinase 1 |
| EMP1 | 7.64E-11 | 3.29E-08 | 7.26 | Up | epithelial membrane protein 1 |
| PAM176C | 4.86E-08 | 1.08E-05 | 7.34 | Up | family with sequence similarity 176, member C |
| ARHGAP15 | 0.000276 | 0.013108 | 7.42 | Up | Rho GTPase activating protein 15 |
| TUBB6 | 1.16E-10 | 4.85E-08 | 7.55 | Up | tubulin, beta 6 class V |
| SLC11A1 | 0.000112 | 0.00649 | 7.6 | Up | natural resistance-associated macrophage protein 1 |
| APOLDI | 6.29E-07 | 9.53E-05 | 7.67 | Up | apolipoprotein L domain containing 1 |
| novel gene | 0.009574 | 0.169235 | 7.78 | Up | Uncharacterized protein |
| ZFP36 | 4.98E-07 | 7.90E-05 | 7.81 | Up | zinc finger protein 36, C3H type, homolog (mouse) |
| RDH10 | 5.11E-11 | 2.40E-08 | 7.83 | Up | retinol dehydrogenase 10 (all-trans) |
| POSL2 | 1.18E-07 | 2.26E-05 | 8.08 | Up | FOS-like antigen 2 |
| CXCL14 | 1.61E-05 | 0.001313 | 8.09 | Up | chemokine (C-X-C motif) ligand 14 |
| IL18RAP | 0.001394 | 1 | 8.3 | Up | Interleukin 18 receptor accessory protein |
| SERPINA1 | 8.38E-08 | 1.68E-05 | 8.31 | Up | serpin peptidase inhibitor, clade A (alpha-1 antiproteinase, antitrypsin), member 1 precursor |
| SELP | 4.19E-06 | 0.00045 | 8.54 | Up | selectin P (granule membrane protein 140kDa, antigen CD62) |
| SERPINB10 | 0.008932 | 1 | 8.59 | Up | serpin peptidase inhibitor, clade B (ovalbumin), member 10 |
| novel gene | 3.27E-06 | 0.000358 | 8.89 | Up | Uncharacterized protein |
| PTHR_CANFA, | 5.05E-05 | 0.00338 | 8.99 | Up | Parathyroid hormone-related protein Osteostatin |
| novel gene | 0.00288 | 0.0777 | 9.35 | Up | Uncharacterized protein |
| TNFSF9 | 7.23E-05 | 0.004613 | 9.35 | Up | tumor necrosis factor (ligand) superfamily, member 9 |
| CLDN1 | 5.61E-07 | 8.68E-05 | 9.45 | Up | claudin 1 |
| ZPLD1 | 3.00E-06 | 0.00033 | 9.51 | Up | zona pellucida-like domain containing 1 |
| RGS2 | 7.16E05 | 0.00459 | 9.68 | Up | regulator of G-protein signaling 2, 24kDa |
| FOS | 1.14E-11 | 5.61E-09 | 9.93 | Up | FBJ murine osteosarcoma viral oncogene homolog |
| SELE | 0.002086 | 0.0631184 | 9.94 | Up | E-selectin precursor |
| novel gene | 1.47E-05 | 0.001215 | 10.16 | Up | Uncharacterized protein |
| IL1RL1 | 2.33E-06 | 0.000273 | 10.38 | Up | interienkin 1 receptor-like 1 |
| CH25H | 1.23E-06 | 0.00016 | 10.44 | Up | cholesterol 25-hydroxylase |
| SELL | 6.69E-07 | 9.98E-05 | 10.52 | Up | selectin L |
| HSD17B13 | 6.67E-08 | 1.44E-05 | 10.53 | Up | hydroxysteroid (17-beta) dehydrogenase 13 |
| CSF2RA | 0.001379 | 0.045712 | 10.55 | Up | colony stimulating factor 2 receptor, alpha, low-affinity (granulocyte-macrophage) |
| CSF3R | 823E-08 | 1.68E-05 | 10.57 | Up | colony stimulating factor 3 receptor (granulocyte) |
| S100P | 0.003185 | 0.040927 | 10.8 | Up | S100 calcium binding protein P |
| CCBP2 | 0.000308 | 0.014374 | 10.92 | Up | chemokine binding protein 2 |
| novel gene | 4.05E-13 | 2.54E-10 | 11.1 | Up | Uncharacterized protein |
| novel gene | 0.000339 | 0.016122 | 11.21 | Up | Uncharacterized protein |
| MYL1 | 1.15E-05 | 0.000976 | 11.51 | Up | myosin, light chain 1, alkali; skeletal, fast |
| U3 | 2.03E.05 | 0.001605 | 11.52 | Up | Small nucleolar RNA U3 |
| DDIT4 | 5.23E-11 | 2.40E-08 | 11.55 | Up | DNA-damage-inducible transcript 4 |
| MAFF | 1.49E-14 | 1.37E-11 | 11.63 | Up | v-maf musculoaponeutrotic fibrosarcoma oncogene homolog F (avian) |
| SLC2A6 | 0,005764 | 0.124788 | 11.72 | Up | solute carrier family 2 (facilitated glucose transporter), member 6 |
| TNC | 0,00114 | 0,039784 | 11.98 | Up | tenascin precursor |
| POSTN | 0.000227 | 0,0 11 507 | 12.43 | Up | periostin, osteoblast specific factor |
| PTGS2 (COX-2) | 1.79E-08 | 4.48E-06 | 12.51 | Up | prostaglandin G/H synthase 2 precursor |
| CD274 | 1.36E-07 | 2.49E-05 | 12.83 | Up | CD274 molecule |
| WNT9B | 1.51E-08 | 3.99E-.06 | 13.34 | Up | wingless-type MMTV integration site family, member 9B |
| ADAMTS4 | 0.002079 | 0,061108 | 13.36 | Up | ADAM metallopeptidase with thrombospondin type 1 motif, 4 |
| CSAR1 | 0.000866 | 0.032018 | 13.47 | Up | C5a anaphylatoxin chemotactic receptor |
| novel gene | 3.22E-07 | 5.48E-05 | 13.63 | Up | Uncharacterized protein |
| ADAMTS9 | 4.44E-16 | 6,12e-13 | 13.83 | Up | ADAM metallopeptidase with thrombospondin type 1 motif. 9 |
| BDNF CANFA | 1.34E-07 | 2.49E-05 | 13.87 | Up | Brain-derived neurotrophic factor |
| GNRH1 | 6.83E-11 | 3.04E-08 | 13.94 | Up | gonadotropin-releasing hormone 1 (lateinizing-releasing hormone) |
| DARC | 3.34E-07 | 5.62E-05 | 14.6 | Up | Duffy blood group, chemokine receptor |
| WFDCI | 5.77E-14 | 4.42E-11 | 15.05 | Up | WAP four-disulfide core domain 1 |
| SLCO2A1 | 7.47E-09 | 2.15E-06 | 15.34 | Up | solute carrier organic anion transporter family member 2A1 |
| novel gene | 3.17E-13 | 2.08E-10 | 15.77 | Up | Uncharacterized protein |
| C19orf59 | 0,000665 | 0.02634 | 16.02 | Up | chromosome 19 open reading frame 59 |
| SLC2A3 | S.22E-14 | 4.23E-11 | 16.51 | Up | solute carrier family 2, facilitated glucose transporter member 3 precursor |
| ILIB_CANFA | 0.00359 | 0.089824 | 16.57 | Up | interleukin-1 beta |
| PLAUR | 5.50E-12 | 3.04E-09 | 17.27 | Up | plasminogen activator, urokinase receptor |
| THBS1 | 0 | 0 | 17.77 | Up | thrombospondin 1 |
| DEISP5 | 1.22E-10 | 4.96E-08 | 18.3 | Up | dual specificity phosphatase 5 |
| S100A9 | 2.81E-13 | 1.93E-10 | 19.31 | Up | S100 calcium binding protein A9 |
| CCL2 | 0 | 0 | 19.56 | Up | C-C motif chemokine 2 precursor |
| ZP2 | 1.32E-06 | 0.000165 | 19.74 | Up | zona pellucida sperm-binding protein 2 precursor |
| FFAR2 | 3.12E-05 | 0.002298 | 20.22 | Up | free fatty acid receptor 2 |
| C15orf48 | 4.55E-05 | 0.003182 | 20.59 | Up | chromosome 15 open reading frame 48 |
| LYSCI_ CANFA | 7.66E-15 | 7.54E-12 | 21.5 | Up | Lysozyme C, milk isozyme |
| RGS1 | 4.93E-14 | 4.33E-11 | 21.69 | Up | regulator of G-protein signaling 1 |
| INHBB | 1.22E-13 | 8.88E-11 | 23.44 | Up | inhibin, beta B |
| Q2EG92_CANFA | 6.83E-12 | 3.55E-09 | 23.73 | Up | Lubricin |
| MT1_CANFA | 3.55E-15 | 3.77E-12 | 24.13 | Up | Metallothionein-1 |
| TESPA1 | 2.91E-05 | 0.002182 | 24.88 | Up | thymocyte expressed, positive selection associated 1 |
| S100A8 | 5.48E-12 | 3.04E-09 | 25.07 | Up | protein S100-A8 |
| novel gene | 2.89E-05 | 0.002 175 | 28.57 | Up | Uncharacterized protein |
| Q7YSA1_CANFA | 0 | 0 | 29.66 | Up | plasminogen activator inhibitor 1 precursor |
| S100A12 | 7.69E-07 | 0.00011 | 29.89 | Up | S100 calcium binding protein A2 |
| BCL2A1 | 2.44E-15 | 2.81E-12 | 32.42 | Up | BCL2-related protein A1 |
| novel gene | 0.003167 | 0.082556 | 33.62 | Up | Uncharacterized protein |
| MIOX | 0.000141 | 0.007811 | 35.1 | Up | myo-inositol oxygenase |
| HAS1 | 1.18E-07 | 2.26E-05 | 37.31 | Up | hyaluronan synthase 1 |
| OSM | 3.94E-05 | 0.002829 | 41.64 | Up | oncostatin M |
| GNATI_CANFA | 0.008282 | 0.155575 | 41.66 | Up | Guanine nucleotide-binding protein G(t) subunit alpha-1 |
| STC1 | 0 | 0 | 41.8 | Up | stanniocalcin 1 |
| QIERY9_CANFA | 9.99E-08 | 1.97E-05 | 42.54 | Up | Uncharacterized protein |
| TIMP1 | 1.03E-06 | 0.000139 | 47.68 | Up | metalloproteinase inhibitor 1 precursor |
| SLC7A5 | 5.36E-13 | 3.22E-10 | 48.85 | Up | solute carrier family 7 (amino acid transporter light chain, L system), member 5 |
| novel gene | 0 | 0 | 50.49 | Up | Uncharacterized protein |
| SLCO4A1 | 2.00E-15 | 2.50E-12 | 50.74 | Up | solute carrier organic anion transporter family, member 4A1 |
| B0FF11_CANFA | 1.05E-09 | 3.70E-07 | 51.17 | Up | Trappin-2; Uncharacterized protein |
| novel gene | 0.005002 | 0.112317 | 53.57 | Up | Uncharacterized protein |
| AQP9 | 0.00899 | 0.057543 | 53.71 | Up | aquaporin 9 |
| Q2LC20_CANFA | 5.74E-08 | 1.26E-05 | 54.31 | Up | Uncharacterized protein |
| HBA_CANFA | 0 | 0 | 58.03 | Up | Hemoglobin subunit alpha |
| PTX3 | 0 | 0 | 58.92 | Up | pentraxin 3, long |
| HBM | 2.83E-09 | 9.53E-07 | 84.56 | Up | hemoglobin, mu |
| AHSP | 4.34E-08 | 9.97E-06 | 100.55 | Up | alpha hemoglobin stabilizing protein |
| MT2_CANFA | 0 | 0 | 134.14 | Up | Metallothionein-2 |
| IL8_CANFA | 0 | 0 | 135.16 | Up | Interleukin-8 |
| MMP8 | 0.000366 | 0.016287 | 162.28 | Up | matrix metallopeptidase 8 (neutrophil collagenase) |
| IL6 | 1.44E-10 | 5.68E-08 | 584.84 | Up | interleukin-6 precursor |
| A7XZY9_CANFA | 0.000243 | 0.012067 | 9999 | Up | Uncharacterized protein |
| FOSL1 | 0.000336 | 0.01537 | 9999 | Up | FOS-like antigen 1 |
| Q6TN20_CANFA | 0.000108 | 0.006318 | 9999 | Up | cathelicidin antimicrobial peptide precursor |
| MMP9 | 0.002261 | 1 | 9999 | Up | matrix metalloproteinase-9 precursor |
| SNORD113 | 0.007908 | 0.150804 | 9999 | Up | Small nucleolar RNA SNORD113/SNORD114 family |
| | | | | | |
| Note: 9999 or -9999 indicates positive or negative infinite number. | | | | | |

The 263 differentially expressed genes in mitral valve tissue are identified in Table 6

**Table 6**

| **Gene name** | **P value** | **FDR** | **Fold change** | **Up/Dn** | **Description** |
|---|---|---|---|---|---|
| IRX4 | 0.0094426 | 0.5521 56 | 137.7129098 | Dn | iroquois homeobox 4 |
| Q8WN71_CANFA | 7.93E-13 | 5.87E-09 | 122.0162934 | Dn | myosin regulatory light chain 2, ventricular/cardiac muscle isoform |
| MYL3 | 1.78E-14 | 2.63E-10 | 36.4518467 | Dn | myosin, light chain 3. alkali: ventricular, skeletal, slow |
| IRK2_CANFA | 0.00010057 | 0.030028 | 26.71429427 | Dn | Inward rectifier potassium channel 2 |
| PABPC1L | 0.00155667 | 0.187433 | 19.26886915 | Dn | poly(A) binding protein, cytoplasmic 1-like |
| IRX3 | 0.00075852 | 0.117131 | 13.42443998 | Dn | iroquois homeobox 3 |
| LRRC38 | 0.00029443 | 0.059732 | 12.63601807 | Dn | leucine rich repeat containing 38 |
| TDRD 1 | 8.79E-05 | 0.028292 | 12.53100309 | Dn | tudor domain containing 1 |
| GPR162 | 4.55E-07 | 0.000613 | 11.88854291 | Dn | G protein-coupled receptor 162 |
| MYOT | 1.81E-07 | 0.000298 | 11.25091164 | Dn | myotilin |
| C3orf43 | 0.00698142 | 0.469977 | 11.06253412 | Dn | chromosome 3 open reading frame 43 |
| O3FAR1 | 0.00810421 | 0.517259 | 9.272574795 | Dn | omega-3 fatty acid receptor 1 |
| LRRC39 | 3.87E-06 | 0.003818 | 8.495742266 | Dn | leucine rich repeat containing 39 |
| ASB18 | 2.87E-06 | 0.003037 | 8.3926 12669 | Dn | ankyrin repeat and SOCS box containing 18 |
| ACT | 5.53E-06 | 0.005117 | 8.072413071 | Dn | AGT angiotensinogen (serpin peptidase inhibitor, clade A, member 8) |
| D PP6 | 8.92E-06 | 0.006135 | 7.818164823 | Dn | dipeptidyl-peptidase 6 |
| RPS11 | 0.00013915 | 0.037469 | 7.52 1 1329 53 | Dn | ribosomal protein S11 |
| EXTL1 | 0.00162694 | 0.193438 | 7.287025302 | Dn | exostoses (multiple)-like 1 |
| CA3 | 9.11E-06 | 0.006135 | 7.130385118 | Dn | carbonic anhydrase 3 |
| HPT_CANFA | 2.44E-07 | 0.000362 | 6.948829584 | Dn | Haptoglobin Haptoglobin alpha chain Haptoglobin beta chain |
| CYP1A1 | 0.0023213 | 0.232988 | 6.847892243 | Dn | cytochrome P450, family 1, subfamily A, polypeptide 1 |
| LAMB4 | 0.00080891 | 1 | 6.631016294 | Dn | laminin, beta 4 |
| Novel gene | 0.00171132 | 0.197093 | 6.404429223 | Dn | Uncharacterized protein |
| SV2B | 0.00633906 | 0.443278 | 5.966893387 | Dn | synaptic vesicle glycoprotein 2B. |
| KCNK13 | 0.00880189 | 0.530726 | 5.20172453 | Dn | potassium channel, subfamily K, member 13 |
| CAPN6 | 0.00063963 | 0.106438 | 5.096581879 | Dn | calpain 6 |
| MYH8_CANFA | 0.00149257 | 0.182686 | 5.082565953 | Dn | Myosin-8 |
| RSPO2 | 0.0002559 | 0.056211 | 4.806677466 | Dn | R-spondin 2 |
| ADIPOQ | 0.00016346 | 0.043228 | 4.798654687 | Dn | adiponectin precursor |
| XIRP2 | 1.50E-05 | 0.008202 | 4.724068879 | Dn | xin actin-binding repeat containing 2 |
| CNKSR2 | 0.00798874 | 1 | 4.607199275 | Dn | connector enhancer of kinase suppressor of Ras 2 |
| VWCE | 0.00252582 | 0.239791 | 4.538860033 | Dn | von Willebrand factor C and EGF domains |
| PAK6 | 0.0052722 | 0.398374 | 4.50185628 | Dn | p21 protein (Cdc42/Rac)-activated kinase 6 |
| Q8MIM4_CANFA | 0.00075926 | 0.117131 | 4.501419438 | Dn | phosphoenolpyruvate carboxykinase, cytosolic |
| HAND1 | 0.00095869 | 0.131464 | 4.463328523 | Dn | heart and neural crest derivatives expressed 1 |
| PLIN1 | 0.00031931 | 0.062223 | 4.391184037 | Dn | perilipin 1 |
| FGF13 | 0.00109213 | 0.14704 | 4.301351408 | Dn | fibroblast growth factor 13 |
| GPD1 | 0.00023118 | 0.053497 | 4.250138926 | Dn | glycerol-3-phosphate dehydrogenase 1 (soluble) |
| Novel gene | 0.00118789 | 0. 155687 | 4.115848845 | Dn | Uncharacterized protein |
| CIDEA | 0.00065272 | 0.106803 | 4.068790504 | Dn | cell death-inducing DFFA-like effector a |
| PLA2G7 | 7.13E-05 | 0.024571 | 3.912769164 | Dn | platelet-activating factor acetylhydrolase |
| RXRG | 0.00040048 | 0.071487 | 3.835168594 | Dn | retinoid X receptor, gamma |
| MAPT | 0.00585597 | 0.421004 | 3.696372113 | Dn | microtubule-associated protein tau |
| ENO3 | 0.00034573 | 0.064003 | 3.600307172 | Dn | enolase 3 (beta, muscle) |
| SMYD2 | 0.00065625 | 0.106803 | 3.580224363 | Dn | SET and MYND domain containing 2 |
| PPP1R1B | 0.00179352 | 0.202763 | 3.401615321 | Dn | protein phosphatase 1, regulatory (inhibitor) subunit 1B |
| KCNJ4 | 0.00113024 | 0.149577 | 3.341648528 | Dn | potassium inwardly-rectifying channel, subfamily J, member 4 |
| ACOT6 | 0.00404691 | 0.331131 | 3.334891906 | Dn | acyl-CoA thioesterase 6 |
| MGST1 | 0.00164756 | 0.193654 | 3.263658261 | Dn | microsomal glutathione S-transferase 1 |
| GPT | 0.00125864 | 0.160728 | 3.196781191 | Dn | glutamic-pyruvate transaminase (alanine aminotransferase) |
| ADSSL1 | 0.00125891 | 0.160728 | 3.111089718 | Dn | adenylosuccinate synthase like 1 |
| Novel gene | 0.00088017 | 0.126663 | 3.014105677 | Dn | Uncharacterized protein |
| MAPK12 | 0.0051303 | 0.38964 | 3.011119574 | Dn | mitogen-activated protein kinase 12 |
| Novel gene | 0.00228645 | 0.232988 | 3.008428356 | Dn | Uncharacterized protein |
| GPR98 | 0.00284501 | 0.260091 | 2.966167357 | Dn | G protein-coupled receptor 98 |
| Novel gene | 0.00163266 | 0.193438 | 2.963721737 | Dn | Uncharacterized protein |
| SLC12A7 | 0.00678406 | 0.46088 | 2.935772145 | Dn | solute carrier family 12 (potassium/chloride transporters), member 7 |
| FABP4 | 0.00310956 | 0.279107 | 2.91333176 | Dn | homolog to human fatty acid binding protein 4, adipocyte |
| TNNT1 | 0.00931 314 | 0.547332 | 2.833529063 | Dn | troponin T type 1 (skeletal, slow) |
| EYA1 | 0.00195033 | 0.213425 | 2.806320921 | Dn | eyes absent homolog 1 (Drosophila) |
| IVNS1ABP | 0.00711125 | 0.474404 | 2.776812953 | Dn | influenza virus NS1A binding protein |
| PDIA2 | 0.00230271 | 0.232988 | 2.688534528 | Dn | protein disulfide isomerase family A. member 2 |
| PPP1R3A | 0.00625535 | 0.442318 | 2.63735175 | Dn | protein phosphatase 1, regulatory subunit 3A |
| HSPB6 | 0.00447266 | 0.352341 | 2.635981052 | Dn | heat shock protein, alpha-crystallin-related, B6 |
| INHBE | 0.00717999 | 0.474623 | 2.620296374 | Dn | inhibin, beta E |
| MRPS6 | 0.00316036 | 0.281957 | 2.617845582 | Dn | mitochondrial ribosomal protein S6 |
| ACSL1 | 0.00396554 | 0.326276 | 2.566513768 | Dn | acyl-CoA synthetase long-chain family member 1 |
| GSTP1 | 0.00497361 | 0.381654 | 2.56311818 | Dn | glutathione S-transferase pi 1 |
| OBSCN | 0.00921911 | 0.543964 | 2.537345537 | Dn | obscurin, cytoskeletal calmodulin and titin-interacting RhoGEF |
| LRRC2 | 0.0097953 | 0.557955 | 2.531793957 | Dn | leucine rich repeat containing 2 |
| FHL2 | 0.00830016 | 0.517259 | 2.510161173 | Dn | four and a half LIM domains 2 |
| OXCT1 | 0.00881556 | 0.530726 | 2.50465171 | Dn | 3-oxoacid CoA transferase 1 |
| MACRODI | 0.00488751 | 0.377 | 2.426051204 | Dn | MACRO domain containing 1 |
| Novel gene | 0.00828926 | 0.517259 | 2.328127388 | Dn | Uncharacterized protein |
| SiR5_CANFA | 0.0071526 | 0.474623 | 2.308794871 | Dn | NAD-dependent protein deacylase sirtuin-5, mitochondrial |
| GMPR | 0.0096464 | 0.554513 | 2.255238481 | Dn | guanosine monophosphate reductase |
| CTSH | 0.00978226 | 0.557955 | 2.31451514 | Up | cathepsin H |
| ELN | 0.00965998 | 0.554513 | 2.375152259 | Up | elastin |
| MTHFDIL | 0.00950707 | 0.552156 | 2.379453069 | Up | methylenetetrahydrofolate dehydrogenase (NADP+ dependent) 1-like |
| LRRC8C | 0.00442286 | 0.352341 | 2.414191301 | Up | leucine rich repeat containing 8 family, member C |
| CSGALNACT1 | 0.00872137 | 0.530726 | 2.464354835 | Up | chondroitin sulfate N-acetylgalactosaminyltransferase 1 |
| B0FF11_CANFA | 0.00362892 | 0.30711 | 2.478539069 | Up | Trappin-2; Uncharacterized protein |
| PMEPA1 | 0.00581774 | 0.420297 | 2.491647533 | Up | prostate transmembrane protein, androgen induced 1 |
| ATP8B1 | 0.00691045 | 0.467323 | 2.514357863 | Up | ATPase, aminophospholipid transporter, class I, type 8B, member 1 |
| CLIC2 | 0.00949909 | 0.5521 56 | 2.559993239 | Up | chloride intracellular channel protein 2 |
| O97702_CANFA | 0.00721068 | 0.474623 | 2.560046473 | Up | integrin beta-3 precursor |
| IER3 | 0.00444912 | 0.352341 | 2.592888197 | Up | immediate early response 3 |
| C13orf33 | 0.00418444 | 0.340503 | 2.597115184 | Up | chromosome 13 open reading frame 33 |
| TEK | 0.00337965 | 0.295017 | 2.613639204 | Up | TEK tyrosine kinase, endothelial |
| NID2 | 0.00761612 | 0.492553 | 2.626788419 | Up | nidogen 2 (osteonidogen) |
| C1QA | 0.00916086 | 0.543964 | 2.639674425 | Up | complement component l, q subcomponent, A chain |
| MYC | 0.00462349 | 0.360389 | 2.667299818 | Up | myc proto-oncogene protein |
| FNDC1 | 0.00913207 | 0.543964 | 2.694018952 | Up | fibromectin type III domain containing 1 |
| SKAP2 | 0.00578608 | 0.420058 | 2.705846502 | Up | src kinase associated phosphoprotein 2 |
| Novel gene | 0.00249457 | 0.239791 | 2.735225921 | Up | Uncharacterized protein |
| SIPR1 | 0.00440559 | 0.352341 | 2.73717941 | Up | sphingosine-1-phosphate receptor 1 |
| CGNL1 | 0.00791609 | 0.509727 | 2.745007327 | Up | cingulin-like 1 |
| Novel gene | 0.00215299 | 0.227756 | 2.746510867 | Up | Uncharacterized protein |
| INPP5D | 0.00875454 | 0.530726 | 2.747558121 | Up | inositol polyphosphate-5-phosphatase, 145kDa |
| CSF1 | 0.00627189 | 0.442318 | 2.761208824 | Up | colony stimulating factor 1 (macrophage) |
| CYTL1 | 0.00728422 | 0.477342 | 2.793202173 | Up | cytokine-like 1 |
| Novel gene | 0.00853615 | 0.522398 | 2.800899076 | Up | Uncharacterized protein |
| MMRN2 | 0.00568962 | 0.420058 | 2.805679081 | Up | multimerin 2 |
| SMOC1 | 0.00347969 | 0.299187 | 2.836437352 | Up | SPARC related modular calcium binding 1 |
| PTGIS | 0.00338642 | 0.295017 | 2.864731645 | Up | prostaglandin 12 (prostacyclin) synthase |
| THBS 1 | 0.00577068 | 0.420058 | 2.872048277 | Up | thrombospondin 1 |
| F13A1 | 0.00195636 | 0.213425 | 2.939233551 | Up | coagulation factor XIII, A1 polypeptide |
| LAPTM5 | 0.00266675 | 0.248393 | 2.945678543 | Up | lysosomal protein transmembrane 5 |
| IGFBP4 | 0.00178576 | 0.202763 | 2.94972406 | Up | insulin-like growth factor binding protein 4 |
| ENPP6 | 0.00292987 | 0.266205 | 2.954901419 | Up | ectonucleotide pyrophosphatase/phosphodiesterase 6 |
| MAFF | 0.00143353 | 0.178408 | 2.956724863 | Up | v-maf musculoaponeurotic fibrosarcoma oncogene homolog F (avian) |
| SNAI1 | 0.00637131 | 0.443278 | 2.978756098 | Up | snail homolog 1 (Drosophila) |
| PTK2B | 0.00275024 | 0.252988 | 2.991066529 | Up | PTK2B protein tyrosine kinase 2 beta |
| CTSS | 0.00237599 | 0.242997 | 3.069428364 | Up | cathepsin S precursor |
| ST6GAL1 | 0.00089001 | 0.126663 | 3.099509659 | Up | ST6 beta-galactosamide alpha-2,6-sialyltranferase 1 |
| STAB1 | 0.00131425 | 0.164949 | 3.104842314 | Up | stabilin 1 |
| A7E3K7_CANFA | 0.00155199 | 0.187433 | 3.116939171 | Up | cytochrome b-245, beta polypeptide |
| MEGF6 | 0.00387037 | 0.321881 | 3.119100417 | Up | multiple EGF-like-domains 6 |
| CCL2 | 0.00089801 | 0.126663 | 3.141187367 | Up | C-C motif chemokine 2 precursor |
| ADAM28 | 0.00920759 | 0.543964 | 3.154759441 | Up | ADAM metallopeptidase domain 28 |
| PKIB | 0.00836025 | 0.518056 | 3.155918612 | U p | protein kinase (cAMP-dependent, catalytic) inhibitor beta |
| WNT11 | 0.00354353 | 0.301608 | 3.167292266 | Up | wingless-type MMTV integration site family, member 11 |
| TNFRSF11B | 0.00097214 | 0.132086 | 3.169642215 | Up | tumor necrosis factor receptor superfamily, member 11 b |
| ICAM1 | 0.00040064 | 0.071487 | 3.173115422 | Up | intercellular adhesion molecule l precursor |
| C1QC | 0.00182952 | 0.205267 | 3.205812393 | Up | complement component l, q subcomponent, C chain |
| MESDC1 | 0.00850967 | 0.522398 | 3.231 558257 | Up | mesoderm development candidate 1 |
| RGS1 | 0.00227737 | 0.232988 | 3.328841086 | Up | regulator of G-protein signaling 1 |
| Novel gene | 0.00033805 | 0.063433 | 3.375005843 | Up | Uncharacterized protein |
| EDN1_CANFA | 0.00349489 | 0.299187 | 3.385314809 | Up | Endothelin-1 |
| Novel gene | 0.00077986 | 0,117854 | 3.387427337 | Up | Uncharacterized protein 1 |
| CISC | 0.00738474 | 0.480629 | 3.398739997 | Up | dipeptidyl peptidase precursor |
| CDH11 | 0.00086943 | 0.126663 | 3.436047547 | Up | cadherin 11, type 2, OB-cadherin (osteoblast) |
| LYVE1 | 0.00026357 | 0.056211 | 3.442746608 | Up | lymphatic vessel endothelial hyaluronan receptor 1 |
| ICOSLG | 0.00195988 | 0.213425 | 3.45146774 | Up | inducible T-cell co-stimulator ligand |
| CD55 | 0.00829165 | 0.517259 | 3.476269663 | Up | CD55 molecule, decay accelerating factor for complement (Cromer blood group) |
| FAM176C | 0.00223235 | 0.232825 | 3.525163079 | Up | family with sequence similarity 176, member C |
| BPI | 0.00127857 | 0.161843 | 3.528487747 | Up | bactericidal/permeability-increasing protein |
| Novel gene | 0.00825134 | 0.517259 | 3.575586735 | Up | Uncharacterized protein |
| DLL1 | 0.0057572 | 0.420058 | 3.638490389 | Up | delta-like 1 (Drosophila) |
| GALNTL2 | 0.00018862 | 0.046558 | 3.640382388 | Up | UDP-N-acetyl-alpha-D-galactosamine:polypeptide N-acetylgalactosaminyltransferase-like 2 |
| BCL3 | 0.00552326 | 0.415226 | 3.667711088 | Up | B-cell CLL/lymphoma 3 |
| Novel gene | 0.00845706 | 0.52181 | 3.667889051 | Up | Uncharacterized protein |
| PTPN6 | 0.0019874 | 0.214842 | 3.678098205 | Up | protein tyrosine phosphatase, non-receptor type 6 |
| Novel gene | 0.006 09549 | 0.436107 | 3.73482333 | Up | Uncharacterized protein |
| PIK3R5 | 0.00384084 | 0.321372 | 3.756710648 | Up | phosphoinositide-3-kinase, regulatory subunit 5 |
| HLA-DQB1 | 0.0016715 | 0.194921 | 3.81425157 | Up | major histocompatibility complex, class II, DQ beta 1 precursor |
| FOXC2 | 0.00227816 | 0.232988 | 3.842512625 | Up | forkhead box C2 (MFH-1, mesenchyme forkhead 1) |
| MT1_ CANFA | 0.00086809 | 0.126663 | 3.868086346 | Up | Metallothionein-1 |
| Novel gene | 0.00027614 | 0.0576 | 3.909109507 | Up | Uncharacterized protein |
| DLA-DRA | 0.00333025 | 0.293577 | 3.921539141 | Up | MHC class II DR alpha chain precursor |
| Novel gene | 0.00044219 | 0.076149 | 3.928340542 | Up | Uncharacterized protein |
| UPSE | 0.00171674 | 0.197093 | 3.930001873 | Up | heparanase |
| CD163 | 0.00023094 | 0.053497 | 3.978735516 | Up | scavenger receptor cysteine-rich type protein M 130 precursor |
| IL10RA | 0.00560792 | 0.419153 | 3.980500931 | Up | interleukin 10 receptor, alpha |
| LCP1 | 0.00040711 | 0.071778 | 4.007048578 | Up | lymphocyte cytosolie protein 1 (L-plastin) |
| SLC27A6 | 0.00739928 | 0.480629 | 4. 012663006 | Up | solute carrier family 27 (fatty acid transporter), member 6 |
| PTPRC | 0.006482 | 0.446504 | 4.015668006 | Up | protein tyrosine phosphatase, receptor type, C |
| SMPDL3A | 0.00060943 | 0.102564 | 4.033996969 | Up | sphingomyelin phoshodiesterase, acid-like 3A |
| AMPN_CANFA | 0.00043757 | 0.076149 | 4.060816961 | Up | Aminopeptidase N |
| ACTG2 | 5.66E-05 | 0.021307 | 4.073051342 | Up | actin, gamma 2, smooth muscle, enteric |
| GATA3 | 0.00451615 | 0.353884 | 4.115791787 | Up | GATA binding protein 3 |
| PDPN | 1.75E-05 | 0.008908 | 4.118417246 | Up | podoplanin precursor |
| ST8S1A6 | 0.00262439 | 0.245995 | 4.134635069 | Up | ST8 alpha-N-acetyl-neuraminide alph.a-2,8-sialyltransferase 6 |
| GADD45B | 0.00076942 | 0.117475 | 4.136899759 | Up | growth arrest and DNA-damage-inducible, beta |
| Novel gene | 4.32E-05 | 0.017766 | 4.146459497 | Up | Uncharacterized protein |
| NPTX2 | 0.00798664 | 0.512044 | 4.171279545 | Up | neuronal pentraxin II |
| Q8SPY1_CANFA | 0.00659035 | 0.451866 | 4.1804261 | Up | leukemia inhibitory factor precursor |
| CD53 | 0.00831247 | 0.517259 | 4.219727979 | Up | CD53 molecule |
| Novel gene | 0.00573048 | 0.420058 | 4.22411 76 | Up | Uncharacterized protein |
| KCNMB1 | 0.00146974 | 0.18139 | 4.234025623 | Up | calcium-activated potassium channel subunit beta-1 |
| Novel gene | 2.29E-05 | 0.010952 | 4.239487876 | Up | Uncharacterized protein |
| Q9TTF1_CANFA | 0.00389039 | 0.321881 | 4.23972297 | Up | T-lymphocyte CD86 activation antigen precursor |
| OLFML2A | 0.00023952 | 0.054573 | 4.240016855 | Up | olfactomedin-like 2A |
| ITGA 10 | 0.00640524 | 0.443278 | 4.247812245 | Up | integrin, alpha 10 |
| CD74 | 0.00092278 | 0.128928 | 4.251052275 | Up | CD74 molecule, major histocompatibility complex, class II invariant chain |
| IGFBP2 | 0.00239923 | 0.237047 | 4.251818462 | Up | insulin-like growth factor binding protein 2, 36kDa |
| CIQB | 0.00011301 | 0.032185 | 4.349742507 | Up | complement component 1, q subcomponent, B chain |
| Novel gene | 0.00011642 | 0.032531 | 4.36581198 | Up | Uncharacterized protein |
| Novel gene | 0.00018478 | 0.046558 | 4.39541687 | Up | Uncharacterized protein |
| WFDC1 | 2.50E-05 | 0.011213 | 4.404688515 | Up | WAP four-disulfide core domain 1 |
| C5AR1 | 0.00345217 | 0.298986 | 4.418081401 | Up | C5a anaphylatoxin chemotactic receptor |
| Novel gene | 7.75E-05 | 0.026077 | 4.425836022 | Up | Uncharacterized protein |
| STC1 | 9.41E-05 | 0.029658 | 4.43867836 | Up | stanniocalcin 1 |
| PROCR | 8.39E-05 | 0.027624 | 4.451433 49 | Up | protein C receptor, endothelial |
| SLC16A3 | 0.00891566 | 0.534579 | 4.520555292 | Up | solute carrier family 1 6, member 3 (monocarboxylic acid transporter 4) |
| ESPN | 0.0021817 | 0.229156 | 4.600912438 | Up | espin |
| CH25H | 0.00093421 | 0.129304 | 4.602762493 | Up | cholesterol 25-hydroxylase |
| NOS3 | 9.74E-06 | 0.00621 | 4.619382465 | Up | nitric oxide synthase, endothelial |
| RELN | 1.72E-05 | 0. 008908 | 4.622040816 | Up | reelin |
| PLEK_CANFA | 0.00299064 | 0.270069 | 4.678285801 | Up | Pleckstrin |
| Q30429_CANFA | 0.00013551 | 0.037166 | 4.777546781 | Up | major histocompability complex, class II, DQ alpha precursor |
| LYPD6 | 0.00066566 | 0.107156 | 4.787823602 | Up | LY6/PLAUR domain containing 6 |
| TFPI2 | 0.00639749 | 0.443278 | 4.811077368 | Up | tissue factor pathway inhibitor 2 |
| Novel gene | 6.11E-05 | 0.022078 | 4.95616155 | Up | Uncharacterized protein |
| THBD | 1.80E-05 | 0.008908 | 5.050093154 | Up | thrombomodulin precursor |
| FOSL2 | 0.00031222 | 0.062223 | 5.144175989 | Up | FOS-like antigen 2 |
| EMCN | 7.37E-06 | 0.005747 | 5.168157368 | Up | endomucin |
| COL23A1 | 0.00624183 | 0.442318 | 5.183224923 | Up | collagen, type XXIII, alpha 1 |
| SNORD44 | 0.0043318 | 0.350568 | 5.230721651 | Up | Small nucleolar RNA SNORD44 |
| Serpine 1 PAI-1) | 1.28E-05 | 0.007573 | 5.248008294 | Up | plasminogen activator inhibitor 1 precursor |
| MPEG1 | 0.00026569 | 0.056211 | 5.343814765 | Up | macrophage expressed 1 |
| SERPINA1 | 0.00070603 | 0.112433 | 5.384080573 | Up | serpin peptidase inhibitor, clade A (alpha-1 antiproteinase, antitrypsin), member 1 precursor |
| CCL24_CANFA | 0.00965828 | 0.554513 | 5.515302439 | Up | C-C motif chemokine 24 |
| Q4ZHP8_CANFA | 0.0002516 | 0.056211 | 5.542819394 | Up | Alpha-2A adrenergic receptor |
| Novel gene | 0.00662883 | 0.45241 | 5.622804942 | Up | Uncharacterized protein |
| INHBB | 0.0019282 | 0.213425 | 5.695095364 | Up | inhibin, beta B |
| WNT2 | 0.00232831 | 0.232988 | 5.75868736 | Up p | wingless-type MMTV integration site family member 2 |
| Novel gene | 475E-05 | 0.019 | 5.837618779 | Up | Uncharacterized protein |
| ITGA8 | 3.15E-05 | 0.013728 | 5.941429157 | Up | integrin, alpha 8 |
| PPBP | 0.00124247 | 0.160728 | 6.061479613 | Up | platelet basic protein precursor |
| CLDN1 | 7.12E-06 | 0.005747 | 6.139537587 | Up | claudin 1 |
| CSF2RB | 1.47E-05 | 0.008202 | 6.468225608 | Up | colony stimulating factor 2 receptor, beta, low-affinity (granulocyte-macrophage) |
| HSD17B13 | 0.00010138 | 0.030028 | 6.789427838 | Up | hydroxysteroid (17-beta) dehydrogenase 13 |
| ENTPD3 | 0.00032849 | 0.06318 | 6.815360638 | Up | ectonucleoside triphosphate diphophohydrolase 3 |
| SLCO2A1 | 0.00033837 | 0.063433 | 6.866286234 | Up | solute carrier organic anion transporter family member 2A1 |
| SNORD43 | 0.00274083 | 0.252988 | 7.174011212 | Up | Small nucleolar RNA SNORD43 |
| Novel gene | 0.00247453 | 0.239528 | 7.183116927 | Up | Uncharacterized protein |
| Novel gene | 0.00247453 | 0.239528 | 7.183116927 | Up | Uncharacterized protein |
| Novel gene | 0.00247453 | 0.239528 | 7.183116927 | Up | Uncharacterized protein |
| STOX1 | 0.00018048 | 0.046558 | 7.231926423 | Up | storkhead box 1 |
| MT2_CANFA | 2.44E-05 | 0.011213 | 7.236038079 | Up | Metallothionein-2 |
| ADAMTS4 | 0.00113117 | 0.149577 | 7.450514215 | Up | ADAM metallopeptidase with thrombospondin type 1 motif, 4 |
| SLC2A3 | 0.00019606 | 0.0476 | 7.48721924 | Up | solute carrier family 2, facilitated glucose transporter member 3 precursor |
| SNORD113 | 0.0002881 | 0.059261 | 8.44460534 | Up | Small nucleolar RNA SNORD113/SNORD114 family |
| COL6A5 | 6.03E-08 | 0.000127 | 8.786873961 | Up | collagen, type VI. alpha 5 |
| DDIT4 | 0.00212956 | 0.226897 | 8.84860482 | Up | DNA-damage-inducible transcript 4 |
| 7SK | 0.00251385 | 0.239791 | 9.205333564 | Up | 7SK RNA |
| F5 | 6.36E-05 | 0.022436 | 9.964863203 | Up | coagulation factor V (proaccelerin, labile factor) |
| Q8SPQ9_CANFA | 5.27E-10 | 1.56E-06 | 9.987682777 | Up | prostaglandin G/H synthase 2 precursor |
| COL13A1 | 0.00512841 | 0.38964 | 1 0.18419469 | Up | collagen, type XIII, alpha 1 |
| PLSCR5 | 1.34E-06 | 0.00155 | 1049150688 | Up | phospholipid scramblase family, member 5 |
| PTX3 | 6.54E-06 | 0.0057 | 10.63398182 | Up | pentraxin 3, long |
| Novel gene | 0.00240089 | 0.237047 | 11.18947157 | Up | Uncharacterized protein |
| SLCO4A1 | 4.93E-05 | 0.019213 | 11.29286782 | Up | solute carrier organic anion transporter family, member 4A1 |
| CSF3R | 0.00088686 | 0.126663 | 11.4548765 | Up | colony stimulating factor 3 receptor (granulocyte) |
| S100A9 | 0,00018626 | 0.046558 | 12.26469966 | Up | S100 calcium binding protein A9 |
| SELP | 3.49E-09 | 8.63E-06 | 11.74032345 | Up | selectin P (granule membrane protein 140kDa, antigen CD62) |
| CHGB | 0.00203775 | 0.218689 | 13.38077711 | Up | chromogranin B (secretogranin 1) |
| OLR1 | 0.0056321 | 0.419153 | 13.39441808 | Up | oxidized low density lipoprotein (lectin-like) receptor 1 |
| REG 3A | 0.00010583 | 0.030731 | 13.94900199 | Up | pancreatitis-associated protein precursor |
| Novel gene | 9.76E-05 | 0.030028 | 14.33648379 | Up | Uncharacterized protein |
| IL6 | 0.00072885 | 0.114832 | 14.38513086 | Up | interleukin-6 precursor |
| 5_8S_rRNA | 8.39E-06 | 0.006135 | 16.57400089 | Up | 5.85 ribosomal RNA |
| IL1RL1 | 4.18E-05 | 0.017697 | 17.141827 | Up | interleukin 1 receptor-like 1 |
| HBA_CANFA | 1.54E-10 | 5.71E-07 | 17.56011028 | Up | Hemoglobin subunit alpha |
| Novel gene | 0.00823939 | 0.517259 | 17.67722609 | Up | Uncharacterized protein |
| Novel gene | 2.84E-12 | 1.40E-08 | 17.82450367 | Up | Uncharacterized protein |
| IL8_CANFA | 1.01E-05 | 0.00621 | 18.31114372 | Up | Interleukin-8 |
| REG3A | 0.00026277 | 0.056211 | 19.76204032 | Up | pancreatitis-associated protein precursor |
| QIERY9_CANFA | 7.59E-08 | 0.000141 | 20.95423577 | Up | Uncharacterized protein |
| WNT9B | 0.00049741 | 0.084673 | 22.13810762 | Up | wingless-type MMTV integration site family, member 9B |
| Novel gene | 0.00707454 | 0.47409 | 24.54691813 | Up | Uncharacterized protein |
| CNTNAP4 | 0.00443392 | 0.352341 | 25.75623834 | Up | contactin associated protein-like 4 |
| Novel gene | 0.00380854 | 0.32048 | 27.06769252 | Up | Uncharacterized protein |
| A7XZY9_CANFA | 0.00083327 | 0.124654 | 28.55536658 | Up | Uncharacterized protein |
| Y_RNA | 0.00478219 | 0.370808 | 37.34284656 | Up | Y RNA |
| Y_RNA | 0.00031522 | 0.062223 | 37.58955679 | Up | Y RNA |
| Y_RNA | 5.75E-05 | 0.021307 | 39.59784465 | Up | Y RNA |
| UPK1B | 0.00039738 | 0.071487 | 40.42343457 | Up | uroplakin 1B |
| Novel gene | 1.36E-06 | 0.00155 | 53.64233007 | Up | U ncharacterized protein |
| SNORD49 | 0.00019959 | 0.047676 | 71.13610116 | Up | Small nucleolar RNA SNORD49 |
| MUC16 | 0.00907706 | 1 | 90.1 5964938 | Up | mucin 16, cell surface associated |
| Novel gene | 0.00323603 | 0.28698 | 151.3736203 | Up | Uncharacterized protein |

The gene expression differentially expressed in both left ventricle and mitral valve tissue are identified in Table 7.

**Table 7**

| **Gene sym** | **Description** |
|---|---|
| A7XZY9 CANFA | Uncharacterized protein |
| ACSL1 | acyl-CoA synthetase long-chain family member 1 |
| ADA MTS4 | ADAM metallopeptidase with thrombospondin type 1 motif, 4 |
| ASB18 | ankyrin repeat and SOCS box containing 18 |
| B0FF11_CANFA | Trappin-2; Uncharacterized protein |
| BCL3 | B-cell CLL/lymphoma 3 |
| C13orf33 | chromosome 13 open reading frame 33 |
| C1QA | complement component 1, q subcomponent, A chain |
| C1QB | complement component 1, q subcomponent, B chain |
| C1QC | complement component 1, q subcomponent, C chain |
| C5AR1 | C5a anaphylatoxin chemotactic receptor |
| CA3 | carbonic anhydraxe 3 |
| CCL2 | C-C motif chemokine 2 precursor |
| CCL24 CANFA | C-C motif chemokine 24 |
| CD163 | scavenger receptor cysteine-rich type 1 protein M130 precursor |
| CH25H | cholesterol 25-hydroxylase |
| ClDEA | cell death-inducing DFFA-like effector a |
| CLDN1 | claudin 1 |
| CLIC2 | chloride intracellular channel protein 2 |
| CSF1 | colony stimulating factor 1 (macrophage) |
| CSF3R | colony stimulating factor 3 receptor (granulocyte) |
| CSGALNACT1 | chondroitin sulfate N-acetylgaladosaminyltransferase 1 |
| CYP1A1 | cytochrome P450, family 1, subfamily A, polypeptide 1 |
| DDIT4 | DNA-damage-inducible transcript 4 |
| DLL1 | delta-like 1 (Drosophila) |
| EMCN | endomucin |
| ENTPD3 | ectonucleoside triphosphate diphosphohydrolase 3 |
| EXTL1 | exostoses (multiple)-like 1 |
| EYA1 | eyes absent homolog 1 (Drosophila) |
| FAM176C | family with sequence similarity 176, member C |
| FNDC1 | fibronectin type III domain containing 1 |
| FOSL2 | FOS-like antigen 2 |
| GADD45B | growth arrest and DNA-damage-inducible, beta |
| GPD1 | glycerol-3-phosphate dehydrogenase 1 (soluble) |
| GPR162 | G protein-coupled receptor 162 |
| GPT | glutamic-pyruvate transaminase (alanine aminotransferase) |
| HBA CANFA | Hemoglobin subunit alpha |
| HSD17B13 | hydroxysteroid (17-beta) dehydrogenase 13 |
| ICAM1 | intercellular adhesion molecule 1 precursor |
| ICOSLG | inducible T-cell co-stimulator ligand |
| IER3 | immediate early response 3 |
| IGFBP2 | insulin-like growth factor binding protein 2, 36kDa |
| IL1RL1 | interlenkin 1 receptor-like 1 |
| IL8 CANFA | interleukin-8 |
| INHBB | inhibin, beta B |
| KCNK13 | potassium channel, subfamily K, member 13 |
| LRRC38 | leucine rich repeat containing 38 |
| LRRC8C | leucine rich repeat containing 8 family, member C |
| LYVE1 | lymphatic vessel endothelial hyaluronan receptor 1 |
| MACROD1 | MACRO domain containing 1 |
| MAFF | v-maf musculoaponeurotic fibrosarcoma oncogene homolog F (avian) |
| MAPK12 | mitogen-activated protein kinase 12 |
| MAPT | microtubule-associated protein tau |
| MT1_CANFA | Metallothionein-1 |
| MT2_CANFA | Metallothionein-2 |
| MTHFD1L | methytenetetrahydrofolate dehydrogenase (NADP+ dependent) 1-like |
| MYC | myc proto-oncogene protein |
| MYH8_CANFA | Myosin-8 |
| 097702_CANFA | integrin beta-3 precursor |
| OBSCN | obscurin, cytoskeletal calmodulin and titin-interacting RhoGEF |
| OLFML2A | olfactomedin-like 2A |
| PABPC1L | poly(A) binding protein, cytoplasmic 1-like |
| PAK6 | p21 protein (Cdc42/Rac)-activated kinase 6 |
| PDIA2 | protein disulfide isomerase family A, member 2 |
| PIK3R5 | phosphoinositide-3-kinase, regulatory subunit 5 |
| PLEK_ CANFA | Pleckstrin |
| PLIN1 | perilipin 1 |
| PMEPA1 | prostate transmembrane protein, androgen induced 1 |
| PPP1R1B | protein phosphatase 1, regulatory (inhibitor) subunit 1B |
| PPP1R3A | protein phosphatase 1, regulatory subunit 3A |
| PTX3 | pentraxin 3, long |
| Q1ERY9_CANFA | Uncharacterized protein |
| Q6R744_CANFA | nitric oxide synthase, endothelial |
| Q7YSA1_CANFA | plasminogen activator inhibitor 1 precursor |
| Q8SPQ9_CANFA | prostaglandin G/H synthase 2 precursor |
| Q95159_CANFA | Uncharacterized protein |
| Q95J95_CANFA | adiponectin precursor |
| Q95LE4_CANFA | interleukin-6 precursor |
| RGS1 | regulator of G-protein signaling 1 |
| S100A9 | S100 calcium binding protein A9 |
| S1PR1 | sphingosine-1-phosohate receptor 1 |
| SELP | selectin P (granule membrane protein 140kDa, antigen CD62) |
| SERPINA1 | serpin peptidase inhibitor, clade A (alpha-1 antiproteinase, antitrypsin), member 1 precursor |
| SLC12A7 | solute carrier family 12 (potassium/chloride transporters), member 7 |
| SLC16A3 | solute carrier family 16, member 3 (monocarboxylic acid transporter 4) |
| SLC2A3 | solute carrier family 2, facilitated glucose transporter member 3 precursor |
| SLCO2A1 | solute carrier organic anion transporter family member 2A1 |
| SLCO4A1 | solute carrier organic anion transporter family, member 4A1 |
| SNAI1 | snail homolog 1 (Drosophila) |
| SNORD1 13 | Small nucleolar RNA SNORD113/SNORD114 family |
| STC1 | stanniocalcin 1 |
| SV2B | synaptic vesicle glycoprotein 2B |
| TDRD1 | tudor domain containing 1 |
| THBD | thrombomodulin precursor |
| THBS1 | thrombospondin 1 |
| VWCE | von Willebrand factor C and EGF domains |
| WFDC1 | W AP four-disulfide core domain 1 |
| WNT9B | wingless-type MMTV integration site family, member 9B |
| XIRP2 | xin actin-binding repeat containing 2 |

in the specification, there have been disclosed typical preferred embodiments of the invention. Although specific terms are employed, they are used in a genetic and descriptive sense only and not for purposes of limitation. The scope of the invention is set forth in the claims.

## Claims

1. A method for diagnosing chronic valvular disease in an animal comprising:
a. analyzing a biological sample from the animal for the presence of gene expression markers associated with chronic valvular disease; and
b. comparing the amount of gene expression markers identified in the sample to a corresponding amount of the same gene expression markers present in a sample from one or more comparable control animals that do not suffer from chronic valvular disease; and
c. using said comparison to diagnose chronic valvular disease in the animal if the gene expression markers found in the animal's sample are greater than the amount present in the control animal's sample,
wherein the diagnosis is based upon determining if the gene expression markers found in the animal's mitral valve sample are greater compared to the amount present in the control animal's mitral valve sample, wherein the gene expression markers are NOS3, COL6A5 (COL29A1), Serpine1 (PAI-1), and SELP.

2. The method of claim 1 wherein the diagnosis is based upon determining if the gene expression markers found in the animal's mitral valve sample are greater compared to the amount present in the control animal's mitral valve sample, wherein the gene expression markers are NOS3, COL6A5 (COL29A1), Serpine1 (PAI-1), SELP, SLC27A6, EDN1_CANFA, CD74, MYC, MT2_CANFA, IL8_CANFA, and IL6.

3. The method of claim 1 wherein the diagnosis is based upon determining if the gene expression markers found in the animal's mitral valve sample are greater compared to the amount present in the control animal's mitral valve sample, wherein the gene expression markers are NOS3, COL6A5 (COL29A1), Serpine1 (PAI-1), SELP, SLC27A6, EDN1_CANFA, CD74, MYC, MT2_CANFA, IL8_CANFA, IL6, and CLDN1.

4. A method for diagnosing chronic valvular disease in an animal comprising:
a. analyzing a biological sample from the animal for the presence of gene expression markers associated with chronic valvular disease; and
b. comparing the amount of gene expression markers identified in the sample to a corresponding amount of the same gene expression markers present in a sample from one or more comparable control animals that do not suffer from chronic valvular disease; and
c. using said comparison to diagnose chronic valvular disease in the animal if the
gene expression markers found in the animal's sample are differentially expressed compared to the amount present in the control animal's sample, wherein the diagnosis is based upon determining if the gene expression markers NOS3, MMP8, MMP9, TIMP1, NPPA, HOPX, and Serpine1 (PAI-1) found in the animal's left ventricle sample are greater compared to the amount present in the control animal's left ventricle sample, and determining if MMP15 gene expression marker found in the animal's left ventricle sample is lesser compared to the amount present in the control animal's left ventricle sample.

5. The method of claim 4 wherein the diagnosis is based upon determining if the gene expression markers NOS3, MMP8, MMP9, TIMP1, NPPA, HOPX, Serpine1 (PAI-1), LOX, TGFBR2, WNT9B, OSMR, OSM, ELOVL7, MT2_CANFA, MT1_CANFA, STAT3, EDNRB, TAGLN2 , RETN, FFAR2, NFKBIA, TLR4, FOS, JUNB, AQP9, SOAT1, SMAD6, EDNRB, PTGS2 (COX-2), IL8_CANFA, and IL6 found in the animal's left ventricle sample are greater compared to the amount present in the control animal's left ventricle sample, and determining if MMP15 gene expression marker found in the animal's left ventricle sample is lesser compared to the amount present in the control animal's left ventricle sample.

6. The method of claim 4 wherein the diagnosis is based upon determining if the gene expression markers NOS3, MMP8, MMP9, TIMP1, NPPA, HOPX, Serpine1 (PAI-1), LOX, TGFBR2, WNT9B, OSMR, OSM, ELOVL7, MT2_CANFA, MT1_CANFA, STAT3, EDNRB, and TAGLN2 found in the animal's left ventricle sample are greater compared to the amount present in the control animal's left ventricle sample, and determining if MMP15 gene expression marker found in the animal's left ventricle sample is lesser compared to the amount present in the control animal's left ventricle sample.

7. The method of claim 1 or 4 wherein the animal is a companion animal.

8. The method of claim 7 wherein the companion animal is a canine.

## Patentansprüche

1. Verfahren zum Diagnostizieren von chronischer Klappenerkrankung bei einem Tier, umfassend:
a. Analysieren einer biologischen Probe von dem Tier auf das Vorhandensein von Genexpressionsmarkern, die mit chronischer Klappenerkrankung verbunden sind; und
b. Vergleichen der Menge an Genexpressionsmarkern, die in der Probe identifiziert wurden, mit einer entsprechenden Menge der gleichen Genexpressionsmarker, die in einer Probe von einem oder mehreren vergleichbaren Kontrolltieren vorhanden sind, die nicht an chronischer Klappenerkrankung leiden; und
c. Verwenden des Vergleichs zum Diagnostizieren einer chronischen Klappenerkrankung in dem Tier, wenn die Genexpressionsmarker, die in der Probe des Tiers gefunden werden, größer sind als die in der Probe des Kontrolltiers vorhandene Menge,
wobei die Diagnose auf dem Bestimmen beruht, ob die in der Mitralklappenprobe des Tiers gefundenen Genexpressionsmarker größer sind als die in der Mitralklappenprobe des Kontrolltieres vorhandene Menge, wobei die Genexpressionsmarker NOS3, COL6A5 (COL29A1), Serpine1 (PAI-1) und SELP sind.

2. Verfahren nach Anspruch 1, wobei die Diagnose auf dem Bestimmen beruht, ob die in der Mitralklappenprobe des Tiers gefundenen Genexpressionsmarker größer sind als die in der Mitralklappenprobe des Kontrolltieres vorhandene Menge, wobei die Genexpressionsmarker NOS3, COL6A5 (COL29A1), Serpine1 (PAI-1), SELP, SLC27A6, EDN1_CANFA, CD74, MYC, MT2_CANFA, IL8_CANFA und IL6 sind.

3. Verfahren nach Anspruch 1, wobei die Diagnose auf dem Bestimmen beruht, ob die in der Mitralklappenprobe des Tiers gefundenen Genexpressionsmarker größer sind als die in der Mitralklappenprobe des Kontrolltieres vorhandene Menge, wobei die Genexpressionsmarker NOS3, COL6A5 (COL29A1), Serpine1 (PAI-1), SELP, SLC27A6, EDN1_CANFA, CD74, MYC, MT2_CANFA, IL8_CANFA, IL6 und CLDN1 sind.

4. Verfahren zum Diagnostizieren von chronischer Klappenerkrankung bei einem Tier, umfassend:
a. Analysieren einer biologischen Probe von dem Tier auf das Vorhandensein von Genexpressionsmarkern, die mit chronischer Klappenerkrankung verbunden sind; und
b. Vergleichen der Menge an Genexpressionsmarkern, die in der Probe identifiziert wurden, mit einer entsprechenden Menge der gleichen Genexpressionsmarker, die in einer Probe von einem oder mehreren vergleichbaren Kontrolltieren vorhanden sind, die nicht an chronischer Klappenerkrankung leiden; und
c. Verwenden des Vergleichs zum Diagnostizieren einer chronischen Klappenerkrankung im Tier, wenn die in der Probe des Tiers gefundenen Genexpressionsmarker im Vergleich zu der in der Kontrolltierprobe vorhandenen Menge differentiell exprimiert sind, wobei die Diagnose auf dem Bestimmen beruht, ob die Genexpressionsmarker NOS3, MMP8, MMP9, TIMP1, NPPA, HOPX und Serpine1 (PAI-1), die in der linken Ventrikelprobe des Tiers gefunden wurden, größer als die in der linken Ventrikelprobe des Kontrolltieres vorhandenen Menge sind, und dem Bestimmen, ob die Menge der MMP15-Genexpressionsmarker, die in der linken Ventrikelprobe des Tiers gefunden wurde, geringer als die in der linken Ventrikelprobe des Kontrolltieres vorhandenen Menge ist.

5. Verfahren nach Anspruch 4, wobei die Diagnose auf dem Bestimmen beruht, ob die Genexpressionsmarker NOS3, MMP8, MMP9, TIMP1, NPPA, HOPX, Serpine1 (PAI-1), LOX, TGFBR2, WNT9B, OSMR, OSM, ELOVL7, MT2_CANFA, MT1_CANFA, STAT3, EDNRB, TAGLN2, RETN, FFAR2, NFKBIA, TLR4, FOS, JUNB, AQP9, SOAT1, SMAD6, EDNRB, PTGS2 (COX-2), IL8_CANFA und IL6, die in der linken Ventrikelprobe des Tiers gefunden wurden, größer als die in der linken Ventrikelprobe des Kontrolltieres vorhandenen Menge sind, und dem Bestimmen, ob die Menge der MMP15-Genexpressionsmarker, die in der linken Ventrikelprobe des Tiers gefunden wurde, geringer als die in der linken Ventrikelprobe des Kontrolltieres vorhandenen Menge ist.

6. Verfahren nach Anspruch 4, wobei die Diagnose auf dem Bestimmen beruht, ob die Genexpressionsmarker NOS3, MMP8, MMP9, TIMP1, NPPA, HOPX, Serpine1 (PAI-1), LOX, TGFBR2, WNT9B, OSMR, OSM, ELOVL7, MT2_CANFA, MT1_CANFA, STAT3, EDNRB und TAGLN2, die in der linken Ventrikelprobe des Tiers gefunden wurden, größer als die in der linken Ventrikelprobe des Kontrolltieres vorhandenen Menge sind, und dem Bestimmen, ob die Menge der MMP15-Genexpressionsmarker, die in der linken Ventrikelprobe des Tiers gefunden wurde, geringer als die in der linken Ventrikelprobe des Kontrolltieres vorhandenen Menge ist.

7. Verfahren nach Anspruch 1 oder 4, wobei das Tier ein Haustier ist.

8. Verfahren nach Anspruch 7, wobei das Haustier ein Hund ist.

## Revendications

1. Méthode de diagnostic d'une valvulopathie chronique chez un animal, comprenant :
a. l'analyse d'un échantillon biologique de l'animal pour la présence de marqueurs de l'expression de gènes associés à la valvulopathie chronique ; et
b. la comparaison de la quantité de marqueurs de l'expression de gènes identifiés dans l'échantillon à une quantité correspondante des mêmes marqueurs de l'expression de gènes présente dans un échantillon provenant d'un ou plusieurs animaux témoins comparables qui ne souffrent pas de valvulopathie chronique ; et
c. l'utilisation de ladite comparaison pour diagnostiquer une valvulopathie chronique chez l'animal si les marqueurs de l'expression de gènes trouvés dans l'échantillon de l'animal sont en excès par rapport à la quantité présente dans l'échantillon de l'animal témoin,
dans laquelle le diagnostic est basé sur le fait de déterminer si les marqueurs de l'expression de gènes trouvés dans l'échantillon de valve mitrale de l'animal sont en excès par rapport à la quantité présente dans l'échantillon de valve mitrale de l'animal témoin, les marqueurs de l'expression de gènes étant NOS3, COL6A5 (COL29A1), Serpine1 (PAI-1) et SELP.

2. Méthode de la revendication 1 dans laquelle le diagnostic est basé sur le fait de déterminer si les marqueurs de l'expression de gènes trouvés dans l'échantillon de valve mitrale de l'animal sont en excès par rapport à la quantité présente dans l'échantillon de valve mitrale de l'animal témoin, les marqueurs de l'expression de gènes étant NOS3, COL6A5 (COL29A1), Serpine1 (PAI-1), SELP, SLC27A6, EDN1_CANFA, CD74, MYC, MT2_CANFA, IL8_CANFA et IL6.

3. Méthode de la revendication 1 dans laquelle le diagnostic est basé sur le fait de déterminer si les marqueurs de l'expression de gènes trouvés dans l'échantillon de valve mitrale de l'animal sont en excès par rapport à la quantité présente dans l'échantillon de valve mitrale de l'animal témoin, les marqueurs de l'expression de gènes étant NOS3, COL6A5 (COL29A1), Serpine1 (PAI-1), SELP, SLC27A6, EDN1_CANFA, CD74, MYC, MT2_CANFA, IL8_CANFA, IL6 et CLDN1.

4. Méthode de diagnostic d'une valvulopathie chronique chez un animal, comprenant :
a. l'analyse d'un échantillon biologique de l'animal pour la présence de marqueurs de l'expression de gènes associés à la valvulopathie chronique ; et
b. la comparaison de la quantité de marqueurs de l'expression de gènes identifiés dans l'échantillon à une quantité correspondante des mêmes marqueurs de l'expression de gènes présente dans un échantillon provenant d'un ou plusieurs animaux témoins comparables qui ne souffrent pas de valvulopathie chronique ; et
c. l'utilisation de ladite comparaison pour diagnostiquer une valvulopathie chronique chez l'animal si les marqueurs de l'expression de gènes sont différentiellement exprimés par rapport à la quantité présente dans l'échantillon de l'animal témoin, le diagnostic étant basé sur le fait de déterminer si les marqueurs de l'expression de gènes NOS3, MMP8, MMP9, TIMP1, NPPA, HOPX et Serpine1 (PAI-1) trouvés dans l'échantillon de ventricule gauche de l'animal sont en excès par rapport à la quantité présente dans l'échantillon de ventricule gauche de l'animal témoin et de déterminer si le marqueur de l'expression du gène MMP15 trouvé dans l'échantillon de ventricule gauche de l'animal est moindre par rapport à la quantité présente dans l'échantillon de ventricule gauche de l'animal témoin.

5. Méthode de la revendication 4 dans laquelle le diagnostic est basé sur le fait de déterminer si les marqueurs de l'expression de gènes NOS3, MMP8, MMP9, TIMP1, NPPA, HOPX, Serpine1 (PAI-1), LOX, TGFBR2, WNT9B, OSMR, OSM, ELOVL7, MT2_CANFA, MT1_CANFA, STAT3, EDNRB, TAGLN2, RETN, FFAR2, NFKBIA, TLR4, FOS, JUNB, AQP9, SOAT1, SMAD6, EDNRB, PTGS2 (COX-2), IL8_CANFA et IL6 trouvés dans l'échantillon de ventricule gauche de l'animal sont en excès par rapport à la quantité présente dans l'échantillon de ventricule gauche de l'animal témoin et de déterminer si le marqueur de l'expression du gène MMP15 trouvé dans l'échantillon de ventricule gauche de l'animal est moindre par rapport à la quantité présente dans l'échantillon de ventricule gauche de l'animal témoin.

6. Méthode de la revendication 4 dans laquelle le diagnostic est basé sur le fait de déterminer si les marqueurs de l'expression de gènes NOS3, MMP8, MMP9, TIMP1, NPPA, HOPX, Serpine1 (PAI-1), LOX, TGFBR2, WNT9B, OSMR, OSM, ELOVL7, MT2_CANFA, MT1_CANFA, STAT3, EDNRB et TAGLN2 trouvés dans l'échantillon de ventricule gauche de l'animal sont en excès par rapport à la quantité présente dans l'échantillon de ventricule gauche de l'animal témoin et de déterminer si le marqueur de l'expression du gène MMP15 trouvé dans l'échantillon de ventricule gauche de l'animal est moindre par rapport à la quantité présente dans l'échantillon de ventricule gauche de l'animal témoin.

7. Méthode de la revendication 1 ou 4, dans laquelle l'animal est un animal de compagnie.

8. Méthode de la revendication 7, dans laquelle l'animal de compagnie est un canidé.
